# EUROPEAN PATENT SPECIFICATION

(11) **EP 2 770 326 B1**
(45) Date of publication and mention of the grant of the patent: **11.09.2019**
(21) Application number: 13382059.7
(22) Date of filing: 26.02.2013
(51) Int. Cl.: G01N 33/68

(54) **Method for the diagnosis of glaucoma based on the determination of serum protein levels**
Verfahren zur Diagnose eines Glaukoms basierend auf die Bestimmung von Serum-Protein-Spiegel
Procédé de diagnostic du glaucome basé sur la détermination du taux de protéines sériques

(43) Date of publication of application: 27.08.2014
(73) Proprietor: Instituto Oftalmologico Fernandez - Vega, S.L., 33012 Oviedo - Asturias (ES)
(72) Inventor: González-Iglesias, Héctor, E-33012 Oviedo - Asturias (ES); Álvarez Fernández, Lydia, E-33012 Oviedo - Asturias (ES); García Díaz, Montserrat, E-33012 Oviedo - Asturias (ES); Coca-Prados, Miguel, New Haven, CT.06510 (US)
(74) Representative: ABG Intellectual Property Law, S.L.

(56) References cited:
- WO-A1-2011/127616
- US-A1- 2009 018 026
- TEZEL G. ET AL.: "Immunoproteomic analysis of potential serum biomarker candidates in human glaucoma", INVEST. OPHTHALMOL. VIS. SCI., vol. 53, no. 13, 13 November 2012 (2012-11-13), pages 8222-8231, XP055072607,
- TEZEL G.: "A proteomics view of the molecular mechanisms and biomarkers of glaucomatous neurodegeneration", PROG. RETIN. EYE RES., vol. 35, July 2013 (2013-07), pages 18-43, XP055072604,
- GRUS F.H. ET AL.: "Transthyretin and complex protein pattern in aqueous humor of patients with primary open-angle glaucoma", MOL. VIS., vol. 14, 4 August 2008 (2008-08-04), pages 1437-1445, XP002706459,
- BOUHENNI R.A. ET AL.: "Identification of differentially expressed proteins in the aqueous humor of primary congenital glaucoma", EXP. EYE RES., vol. 92, no. 1, January 2011 (2011-01), pages 67-75, XP027577200,
- PIERAGOSTINO D. ET AL.: "Differential protein expression in tears of patients with primary open angle and pseudoexfoliative glaucoma", MOL. BIOSYST., vol. 8, no. 4, April 2012 (2012-04), pages 1017-1028, XP055072611,
- KOKOTAS H. ET AL.: "Biomarkers in primary open angle glaucoma", CLIN. CHEM. LAB. MED., vol. 50, no. 12, December 2012 (2012-12), pages 2107-2119, XP055072609,
- BHATTACHARYA S.K. ET AL.: "Molecular biomarkers in glaucoma", INVEST. OPHTHALMOL. VIS. SCI., vol. 54, no. 1, 7 January 2013 (2013-01-07), pages 121-131, XP055072378,

## Description

### FIELD OF THE INVENTION

The present invention falls within the field of diagnosis and, more specifically, it relates to the diagnosis of glaucoma based on the determination of the serum levels of some proteins. Likewise, the invention also relates to a method for the differential diagnosis of primary open angle glaucoma and pseudoexfoliation glaucoma.

### BACKGROUND OF THE INVENTION

A leading cause of blindness worldwide, glaucoma encompasses a complex group of disorders that are multigenic and multifactorial in origin, but are all characterized by progressive degeneration of the optic nerve, retinal ganglion cell death and the loss of the visual field. Primary open-angle glaucoma (POAG) and pseudoexfoliation glaucoma (PEXG) are among the most prevalent types of glaucoma in developed countries. Whereas an abnormal elevation in the intraocular pressure (IOP) is the best known risk factor associated with the pathogenesis and progression of POAG, the presence of pseudoexfoliation syndrome is linked with PEXG, a secondary form of glaucoma. The projected number of people worldwide with POAG in 2020 has been estimated to reach 60 million. In certain regions of the world, including the northwest of Spain and some provinces of Saudi Arabia, the prevalence of PEXG among pseudoexfoliation syndrome patients can reach up to 30%.

The increased IOP observed is usually associated with a dysfunction of the normal exit of aqueous humor in the eye, due to an alteration in the cell physiology of the trabecular meshwork in POAG, and to the buildup of fibrillar aggregates of material from tissues of the anterior segment of the eye (i.e., iris, lens, ciliary body) in PEXG. Additional clinical differences between POAG and PEXG include: (i) a more elevated IOP in PEXG patients than those with POAG (relative to control); (ii) a lack of an IOP response to steroids among PEXG patients when compared to those with POAG; and (iii) quantitative histological differences of cross-sections of the optic nerve. The frequency of onset and rate of optic neurodegeneration in both POAG and PEXG increase with age, and ultimately lead to blindness. In most cases, degeneration of the optic head precedes detectable field loss, and it has been estimated that the threshold of early glaucoma may imply 25% to 35% loss of retinal ganglion cells by the time early field defects are found.

In spite of the large number of linkage studies, candidate gene reports, and genome-wide-association investigations conducted on POAG and PEXG populations worldwide, only genes with limited population effects have been discovered. Notable results of these studies is that mutations in the gene *Myocilin* have been at various frequencies with POAG, and common sequence variants in the gene *Lysyl oxidase-like 1* are linked with PEXG. In contrast, only limited investigations have been conducted in the field of differential proteomics in search of molecular biomarkers of glaucoma. In particular, the search for biomarkers (i.e., proteins) in readily available biological fluids (those that do not require invasive surgery), such as tears, has yielded discoveries that may be beneficial in the early diagnostic and management of glaucoma.

Tezel G. et al., Invest. Ophtalmol. Vis. Sci., vol. 53, no. 13, 13 November 2012, pages 8222-8231 discloses an immunoproteomic analysis of potential serum biomarker candidates in human glaucoma which follows the same procedure as that disclosed in the present application. However, apolipoprotein A-IV is not comprised in the 10 listed serum protein biomarkers selected for validation in serum of glaucomatous and control samples by specific ELISA. Tezel G., Prog. Retin. Eye Res., vol. 35, July 2013, pages 18-43 is a review reporting on the results of proteomics studies and the identification of the molecular mechanisms and biomarkers of glaucoma in different samples such as tears, blood, aqueous humor and pseudoexfoliative material. However, apolipoprotein A-IV is not mentioned. Grus F.H. et al., Mol. Vis., vol. 14, 4 August 2008, pages 1437-1445 discloses 2D- gel electrophoresis, mass spectrometry and validation by ELISA performed in aqueous humor samples to the identification of elevated levels of transthyretin in aqueous humor of patients with primary open-angle glaucoma. However, apolipoprotein A-IV is not mentioned. Bouhenni R.A. et al., Exp. Eye Res., vol. 92, no. 1, January 2011, pages 67-75 discloses the identification of differentially expressed proteins in the aqueous humor of primary congenital glaucoma by mass spectrometry and western blot and teaches that apolipoprotein A-IV was detected at significantly higher levels in aqueous humor of primary congenital glaucoma patients compared to controls. However, primary open angle glaucoma or pseudoexfoliation glaucoma are not mentioned. Pieragostino D. et al., Mol. Biosyst., vol. 8, no. 4, April 2012, pages 1017-1028 discloses the differential protein expression in tears of patients with primary open angle and pseudoexfoliative glaucoma and teaches that serotransferrin and apolipoprotein A-I are overexpressed in pseudoexfoliation glaucoma and that apolipoprotein A-I is overexpressed in POAG. However, apolipoprotein A-IV is not mentioned.

There are several major obstacles that complicate the discovery of biomarkers. First, it must be determined whether or not medication is influencing the expression of biomarkers in bodily fluids. Recent proteomic studies were successfully carried out with tears and aqueous humor, which included POAG and PEXG patients undergoing treatment for glaucoma. However, it remains untested whether biomarkers in serum proposed for diagnosing untreated glaucoma can be relied upon in patients taking glaucoma medication. Secondly, it is generally believed that tests to validate glaucoma-biomarker candidates should include subjects with different types and stages of glaucoma, as well as patients with other ocular and neurodegenerative diseases.

Therefore, there is still a need in the art for reliable methods useful for diagnosing different types of glaucoma, such as POAG and PEXG, based on the analysis of bodily fluids, which can be easily obtainable by non-invasive means.

### SUMMARY OF THE INVENTION

The inventors of the present invention have observed that, surprisingly, patients suffering from glaucoma exhibit different expression levels of the serum proteins of Table 1 when compared with control subjects. In particular, a study based on the identification by gel electrophoresis (2D-DIGE (Bidimensional - Difference gel electrophoresis) followed by mass spectrometry analysis, of 35 serum proteins whose expression was altered in patients with glaucoma compared with control subjects was conducted. Seventeenth of these 35 proteins were validated by ELISA determination of serum concentrations, showing significant differences in the levels of thirteen of these proteins between subjects with glaucoma (POAG or PEXG) and control subjects (Table 5). Naive Bayes machine learning models generated from these data for each of the individual biomarkers showed AUC values in all cases greater than 0.5, and above 0.8 for many of the biomarkers, proving the utility of each of these protein as individual biomarkers (Table 6). Further, the ELISA-determined serum concentration data of the top-17-ranked out of said 35 proteins were analyzed by "Stepwise discriminant analysis" in order to determine the variables that have a greater power of discrimination. By means of this methodology, different panels suitable for diagnosing different types of glaucoma were identified; among them, a panel of 6 proteins (apolipoprotein A-IV, serotransferrin, complement C3, apolipoprotein L1, complement C4 and Ig gamma-2 chain C region) was identified that was used for generating predictive multivariable models whose results showed that the determination of the serum levels of these biomarkers allowed obtaining correct assignment values of 81%, with a specificity of 83% and an sensitivity of 92% (see Example 1). Furthermore, taking into consideration the presence or absence of pseudoexfoliation syndrome in the subject the determination of the serum level of some of the proteins contained in said biomarker panel allowed a correct discrimination between groups of patients with primary open-angle glaucoma (POAG) and pseudoexfoliation glaucoma (PEXG) in 86% of the patients.

Based on the previous findings, the following inventive aspects have been developed.

In an aspect, the invention relates to an *in vitro* method for the diagnosis of a glaucoma selected from primary open angle glaucoma and pseudoexfoliation glaucoma in a subject comprising (i) determining in a sample from said subject the expression level of apolipoprotein A-IV, and (ii) comparing the expression level of apolipoprotein A-IV with a reference value, wherein (a) if the subject suffers from pseudoexfoliation syndrome, an increase in the expression level of apolipoprotein A-IV compared to a reference value is indicative of pseudoexfoliation glaucoma, or (b) if the subject does not suffer from pseudoexfoliation syndrome, an increase in the expression level of apolipoprotein A-IV compared to a reference value is indicative of primary open angle glaucoma.

In another aspect, the invention relates to a kit as defined in appended claims 11-13.

In another aspect, the invention relates to the use of a kit as defined in claims 14-15 for diagnosis of a glaucoma selected from primary open angle glaucoma and pseudoexfoliation glaucoma.

### DETAILED DESCRIPTION OF THE INVENTION

### Method for the diagnosis of glaucoma

Thus, in a first aspect, the invention relates to an *in vitro* method for the diagnosis of glaucoma selected from primary open angle glaucoma and pseudoexfoliation glaucoma in a subject, hereinafter referred to as "diagnostic method of the invention", comprising
(i) determining in a sample from said subject the expression level of apolipoprotein A-IV and optionally of at least one biomarker selected from the biomarkers of Table 1, and
(ii) comparing the expression levels of apolipoprotein A-IV and optionally of said at least one biomarker with its reference value,
wherein Table 1 is

**Table 1**

| **Protein name** | **Gene name** |
|---|---|
| Apolipoprotein A-IV | APOA4 |
| Complement C3 | C3 |
| Serotransferrin | TF |
| Vitronectin | VTN |
| Transthyretin | TTR |
| Alpha-1 antitrypsin | SERPINA1 |
| Fibulin-1 | FBLN1 |
| Apolipoprotein A1 | APOA1 |
| Ficolin-3 | FCN3 |
| Complement factor H | CFH |
| Inter-alpha-trypsin inhibitor heavy chain H4 | ITIH4 |
| Apolipoprotein L1 | APOL1 |
| Serum albumin | ALB |

and, wherein,
a) if the subject suffers from pseudoexfoliation syndrome, an increase in the expression level of apolipoprotein A-IV and optionally of said at least one biomarker compared to a reference value is indicative of pseudoexfoliation glaucoma, or
b) if the subject does not suffer from pseudoexfoliation syndrome, an increase in the expression level of apolipoprotein A-IV and optionally of at least one biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, and apolipoprotein L1 compared to a reference value, and/or a decrease in the expression level of serum albumin compared to a reference value is indicative of primary open angle glaucoma.

The term "glaucoma", as used herein, refers to a group of eye conditions that lead to damage to the optic nerve, in most cases due to increased pressure in the eye or high intraocular pressure. The term glaucoma encompasses open-angle (chronic) glaucoma, angle-closure (acute) glaucoma, pseudoexfoliation glaucoma, congenital glaucoma and secondary glaucoma. In most of the cases, the increased intraocular pressure is due to a dysfunction of the normal exit of aqueous humor in the eye.

The term "primary open angle glaucoma" or "POAG", as used herein, refers to the most common type of glaucoma. POAG is characterized by progressive and slowly increase in the eye pressure over time accompanied by loss of peripheral vision or tunnel vision. Advanced POAG can lead to blindness. The increase in intraocular pressure is caused by blockage of the trabecular meshwork, which normally drains the aqueous humor from the eye.

The term "pseudoexfoliation glaucoma" or "PEXG", as used herein, refers to a type of glaucoma caused by the deposition of a protein-like material within the anterior segment of the eye. Pseudoexfoliation syndrome (PEX) is considered a major risk factor for the development of secondary open angle glaucoma. In contrast to POAG, PEXG disease runs a more aggressive clinical course with high IOP at onset, faster rates of progression, poor response to medical therapy, and increased need for surgical intervention.

The expression "method for the diagnosis of glaucoma", as used herein, refers to a method that consists essentially of the steps of the diagnostic method of the invention although it may include additional steps. "Diagnosing", as used herein, refers to evaluating the probability according to which a subject suffers from a disease, in particular from glaucoma. The method for the diagnosis of glaucoma of the invention is carried out *in vitro.* The term *"in vitro",* as used herein, means that it is no not performed over the human or animal body.

The term "subject", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race.

The first step of the diagnostic method of the invention involves the determination of de expression levels of apolipoprotein A-IV and optionally of at least one biomarker selected from the biomarkers of Table 1 in a sample from a subject.

The term "sample", as used herewith, refers to any sample collected from a subject, in which the markers of the methods of the invention can be measured. Suitable samples for use in the present invention include any bodily fluid.

In a preferred embodiment, the sample is a bodily fluid sample. The term "bodily fluid sample", as used herewith, refers to a sample of a liquid derived from the body of a living organism. Illustrative, non-limitative, examples of bodily fluids include tears, blood, plasma or blood serum. Preferably, the bodily fluid sample is blood, plasma or blood serum.

The term "biomarker", as used herewith, refers to a protein the occurrence or amount of which is characteristic for a specific situation, for example, a disease such as glaucoma.

The biomarkers useful for the method of diagnosis of the invention are those proteins included in Table 1.

The term "apolipoprotein A-IV" or "apoA-IV", as used herein, refers a protein with in humans is the product of the *APOA4* gene. The primary translation product of the *APOA4* gene is a 396-residue preprotein, which undergoes proteolytic processing to yield apoA-IV, a 376-reside mature O-linked glycoprotein, which is secreted into circulation on the surface of newly synthesized chylomicron particles. The apoA-IV can be from any origin, for example human, bovine, murine, equine, canine, etc, depending on the subject, which is going to be diagnosed with the diagnostic method of the invention. In a particular embodiment, the apoA-IV is the human protein with the UniProt accession number P06727 (November 28, 2012).

The term "complement C3" or "C3", as used herein, refers to a protein of the complement system which plays a central role in both classical and alternative complement activation. In humans, complement C3 is encoded by the *C3* gene. The complement C3 can be from any origin, for example human, bovine, murine, equine, canine, etc, depending on the subject which is going to be diagnosed with the diagnostic method of the invention. In a particular embodiment, the complement C3 is the human protein with the UniProt accession number P01024 (November 28, 2012).

The term "serotransferrin" or "transferrin" or "siderophilin" or "TF" as used herein, refers to an iron-binding blood plasma glycoprotein that controls the level of free iron in biological fluids. In humans, serotransferrin is encoded by the *TF* gene. The TF can be from any origin, for example human, bovine, murine, equine, canine, etc, depending on the subject which is going to be diagnosed with the diagnostic method of the invention. In a particular embodiment, the TF is the human protein with the UniProt accession number P02787 (November 28, 2012).

The term "vitronectin" or "VTN", as used herein, refers to a glycoprotein found in serum and in the extracellular matrix that promotes cell adhesion and spreading. It is a member of the pexin family. In humans, vitronectin is encoded by the *VTN* gene. The vitronectin can be from any origin, for example human, bovine, murine, equine, canine, etc., depending on the subject which is going to be diagnosed with the diagnostic method of the invention. In a particular embodiment, the vitronectin is the human protein with the UniProt accession number P04004 (November 28, 2012).

The term "transthyretin" or "TTR", as used herein, refers to a serum and cerebrospinal fluid carrier of the thyroid hormone thyroxine and of vitamine A. In humans transthyretin is encoded by the gen *TTR.* The transthyretin can be from any origin, for example human, bovine, murine, equine, canine, etc, depending on the subject which is going to be diagnosed with the diagnostic method of the invention. In a particular embodiment, the transthryretin is the human protein with the UniProt accession number Q53WY6 (November 28, 2012).

The term "alpha-1 antitrypsin" or "serpin A1" or "SERPINA1", as used herein, refers to a protease inhibitior belonging to the serpin superfamily. In humans alpha-1 antitrypsin is encoded by the gene *SERPINA1*. The alpha-1 antitrypsin can be from any origin, for example human, bovine, murine, equine, canine, etc, depending on the subject which is going to be diagnosed with the diagnostic method of the invention. In a particular embodiment, the alpha-1 antitrypsin is the human protein with the UniProt accession number P01009 (November 28, 2012).

The term "fibulin-1" or "FBLN1", as used herein, refers to a secreted glycoprotein than is found in association with extracellular matrix structures including fibronectin and elastin containing fibers and basement membrane and in blood associated with fibrinogen. In humans, fibulin-1 is encoded by *FBLN1* gene. The fibulin-1 can be from any origin, for example human, bovine, murine, equine, canine, etc, depending on the subject which is going to be diagnosed with the diagnostic method of the invention. In a particular embodiment, the fibulin-1 is the human protein with the UniProt accession number P23142 (November 28, 2012).

The term "apolipoprotein A-I" or "apoA-I" or "APOA1", as used herein, refers to an apoprotein that is the main component of high density lipoprotein (HDL) in plasma. In humans, apoA-I is encoded by the gene *APOA1*. The apoA-I can be from any origin, for example human, bovine, murine, equine, canine, etc, depending on the subject which is going to be diagnosed with the diagnostic method of the invention. In a particular embodiment, the apoA-I is the human protein with the UniProt accession number P02647 (November 28, 2012).

The term "ficolin-3" or FCN3", as used herein, refers to a member of the family of proteins ficolins which consists of a collagen-like domain and a fibrinogen-line domain. In humans, ficolin-3 is encoded by the *FCN3* gene. The ficolin-3 can be from any origin, for example human, bovine, murine, equine, canine, etc, depending on the subject which is going to be diagnosed with the diagnostic method of the invention. In a particular embodiment, the ficolin-3 is the human protein with the UniProt accession number O75636 (November 28, 2012).

The term "complement factor H" or "CFH", as used herein, refers to a plasma glicoprotein which act as a regulator of the alternative pathway of the complement system. The complement factor H can be from any origin, for example human, bovine, murine, equine, canine, etc, depending on the subject which is going to be diagnosed with the diagnostic method of the invention. In a particular embodiment, the complement factor H is the human protein with the UniProt accession number P08603 (November 28, 2012).

The term "inter-alpha-trypsin inhibitor heavy chain H4" or "ITIH4", as used herein, refers to heavy chain which can form part of the plasma proteins inter-alpha-trypsin inhibitors together with a light chain selected from the group AMBP or SPINT2. In humans, it is encoded by the *ITIH4* gene. The inter-alpha trypsin inhibitor heavy chain H4 can be from any origin, for example human, bovine, murine, equine, canine, etc, depending on the subject which is going to be diagnosed with the diagnostic method of the invention. In a particular embodiment, the inter-alpha trypsin inhibitor heavy chain H4 is the human protein with the UniProt accession number Q14624 (November 28, 2012).

The term "apolipoprotein L1" or "apoL-I" or "APOL1", as used herein, refers to a minor apoprotein component of High-density lipoprotein (HDL) which is synthesized in the liver and also in many other tissues, including pancreas, kidney, and brain. In humans, apoL-I is encoded by the *APOL1* gene. The apoL-I can be from any origin, for example human, bovine, murine, equine, canine, etc, depending on the subject which is going to be diagnosed with the diagnostic method of the invention. In a particular embodiment, the apoL-I is the human protein with the UniProt accession number 014791 (November 28, 2012).

The term "serum albumin" or "albumin" or "ALB", as used herein, refers to a globular protein that in humans is encoded by the *ALB* gene. Serum albumin, which is the most abundant plasma protein in mammals, regulates the colloidal osmotic pressure of blood. The serum albumin can be from any origin, for example human, bovine, murine, equine, canine, etc, depending on the subject which is going to be diagnosed with the diagnostic method of the invention. In a particular embodiment, the serum albumin is the human protein with the UniProt accession number P02768 (February 6, 2013).

The inventors have also found that the expression levels of other proteins are also altered in glaucoma patients compared with control subjects. The determination of the expression levels of these additional biomarkers could also be useful to aid in the diagnosis of a glaucoma selected from POAG and PEXG. Therefore, in a particular embodiment, the diagnostic method of the invention further comprises determining the expression level of one or more biomarkers selected from the group consisting of Ig gamma-2 chain C region, antithrombin-III, complement C4A, serum amyloid P-component and combinations thereof.

The term "antithrombin III" or "SERPINC1", as used refers to a serine protease inhibitor in plasma that regulates the blood coagulation cascade. In humans antithrombin III is encoded by the *SERPINC1* gene. The antithrombin III can be from any origin, for example human, bovine, murine, equine, canine, etc, depending on the subject which is going to be diagnosed with the diagnostic method of the invention. In a particular embodiment, the antithrombin III is the human protein with the UniProt accession number P01008 (January 9, 2013).

The term "Ig gamma-2 chain C region" or "G2m marker" or "IGHG2", as used herein, refers to a protein that in humans is encoded by the *IGHG2* gene. The IGHG2 can be from any origin, for example human, bovine, murine, equine, canine, etc, depending on the subject which is going to be diagnosed with the diagnostic method of the invention. In a particular embodiment, the IGHG2 is the human protein with the UniProt accession number P01859 (November 28, 2012).

The term "complement C4-A" or "C4A", as used herein, refers to a protein of the complement system which plays a central role in the activation of the classical pathway of the complement system. In humans, C4A is encoded by the *C4A* gene. The C4A can be from any origin, for example human, bovine, murine, equine, canine, etc, depending on the subject which is going to be diagnosed with the diagnostic method of the invention. In a particular embodiment, the C4A is the human protein with the UniProt accession number P0C0L4 (November 28, 2012).

The term "serum amyloid P-component" or "APCS", as used herein, refers to a protein which in humans is encoded by the *APCS* gene. The serum amyloid P-component can be from any origin, for example human, bovine, murine, equine, canine, etc, depending on the subject which is going to be diagnosed with the diagnostic method of the invention. In a particular embodiment, the serum amyloid P-component is the human protein with the UniProt accession number P02743 (January 9, 2013).

The diagnostic method of the invention involves the determination of the expression level of the biomarkers mentioned above. The term "expression level", as used herein, refers to the quantity of a protein detectable in a sample. The methods for determining the level of the biomarkers according to the present invention can be based on assaying the level of expression of protein in the sample as a whole, preferably in the non-cellular fraction of the sample. The determination of the biomarkers can be carried out by any method suitable for the detection and quantification of a protein and the person skilled in the art is familiar with said methods. By way of a non-limiting illustration, the expression level of a biomarker at a protein level can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragment thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known test that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays. In a preferred embodiment of the invention, the determination of the levels of biomarkers is carried out by an immunological method. As used herein, "immunological method", when applied to a determination, relates to any method which involves the use of one or more antibodies specific for a target substance in order to determine the amount/concentration of said target substance excluding other substances found in the sample. Suitable immunological methods include, without limitation, Western blot, immunoprecipitation, enzyme-linked immunosorbent assay (ELISA), surface plasmon resonance, radioimmunoassay (RIA). In a preferred embodiment, the determination of the levels of biomarkers is carried out by ELISA or quantum dot-based multiplex assays.

The term "ELISA" or "enzyme-linked immunosorbent assay", as used herein, stands for enzyme-linked immunosorbent assay and relates to an assay by which an unknown amount of target substance (the biomarkers of the diagnostic method of the invention) is affixed to a surface, and then a specific antibody is washed over the surface so that it can bind to the antigen. This antibody is linked to an enzyme, and in the final step a substance is added that the enzyme can convert to some detectable signal. Different types of ELISA assays are known and can be applied to the method of the invention, including direct ELISA, sandwich ELISA, competitive ELISA and ELISA reverse method & device (ELISA-R m&d).

Direct ELISA is carried out by contacting the test sample comprising the biomarkers with a solid support, which has been previously coated with a concentrated solution of a non-interacting protein or reagent (bovine serum albumin, casein). Once the biomarkers present in the test sample is absorbed onto the support, an antibody specific for the biomarker is added under conditions adequate for binding onto the biomarker. The antibody, which is bound is then detected with a secondary antibody which is coupled to a detectable tag or to a substrate modifying enzyme. The signal resulting from the detectable tag or from the substrate is then proportional to the amount of antibody bound to the support, which, in turn, correlates directly with the amount of biomarker in the sample.

Competitive ELISA assay includes a diagnostic step wherein the test sample comprising an unknown amount of biomarker is contacted with a first antibody as defined above. The antibody-antigen complexes are added to an antigen coated well. Once the support is washed to remove any non-specifically bound complexes, the amount of first antibody is detected with a second antibody, which is coupled to a detectable moiety. In this type of assays, the higher the original antigen concentration, the weaker the eventual signal. An alternative competitive ELISA assay is that which includes an enzyme-linked antigen rather than enzyme-linked antibody. The labeled antigen competes for primary antibody binding sites with the sample antigen (unlabeled). Using this type of assays, the concentration of antigen in the sample results inversely correlates with the amount of labeled antigen retained in the well and, accordingly, in a weaker signal.

ELISA reverse method & device (ELISA-R m&d) uses an innovative solid phase constituted of an immunosorbent polystyrene rod with 4-12 protruding ogives; the entire device is suitable to be introduced in a test tube containing the collected sample and the following steps (washing, incubation in conjugate and incubation in chromogenous) are easily carried out by immerging the ogives in microwells of standard microplates prefilled with reagents, sealed and stored until their use.

The ELISA sandwich assay involves coating a support with a first antibody specific for the biomarker, applying the sample containing the biomarker which will result in the binding of the biomarker to the first antibody and applying a second antibody also specific for the biomarker, wherein said second antibody is usually coupled to a detectable tag or to a substrate-modifying enzyme. The signal generated by the tag or by the converted substrate is the proportional to the amount of antigen in the sample.

The term "quantum dots" refers to semiconductor nanoparticles with unique optical and electronic properties, in particular, their reduce tendency to photobleach and the dependence of their fluorescence wavelength on their size which make them suitable for fluorescent probing of biomarkers. The biomarkers of the diagnostic method of the invention can be detected by means of antibody-linked quantum dots. In a particular embodiment, the biomarkers according to the diagnostic method of the invention are detected by a multiplex analysis based on antibody-linked quantum dots.

Alternatively, the expression level of the biomarkers according to the diagnostic method of the invention can be determined by mass spectrometry. In a particular embodiment, the expression level of the biomarkers according to the diagnostic method of the invention is determined by multiple reaction monitoring (MRM). The term "multiple reaction monitoring" or "MRM", as used herein, refers to a method of detection of multiple ions from one or more precursor ions in quantitative tandem mass spectrometry. MRM assay configuration starts with the selection of "signature" peptides that act as surrogates for the quantification of protein of interest. Peptide selection is based upon experimental observation using commercially available standards. These signature peptides are synthesized as stable isotope-labeled internal standards. MRM assays are based on the selection of specific enzimatically digested peptides as stoichiometric representatives of the proteins from which they are cleaved. Samples must be enzymatically digested by means of a "digestive enzyme", which is an enzyme capable of cleaving or hydrolyzing peptides or proteins into fragments in either a specific or generic, random manner. Digestive enzymes include proteases such as trypsin, papain, endoproteinase LysC, endoproteinase ArgC, staph aureus V8, chymotrypsin, Asp-N, Asn-C, pepsin, and endoproteinase GluC. The tryptic peptides are quantitated against a spiked internal standard (a synthetic stable isotope-labeled peptide) to yield a measure of protein concentration.

The second step of the diagnostic method of the invention comprises comparing the expression level of each of the biomarkers with its reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The reference value according to the diagnostic method of the invention is obtained from one or more subjects who do not suffer from glaucoma (i.e., control subjects).

According to the diagnostic method for the invention, a subject is considered that does not suffer from glaucoma if said subject does not meet the diagnostic criteria for glaucoma, namely, the subject does not present characteristic optic-disc damage with the corresponding characteristic changes in the visual field and the presence of open anterior chamber angle. In a particular embodiment, the subjects from whom the reference value according to the diagnostic method of the invention is obtained do not suffer from any relevant ocular pathology, such as clinically detectable inflammation, infection, retinopathies or maculopathies. In a particular embodiment, one or more of the subjects from whom the reference value according to the diagnostic method of the invention is obtained may suffer from cataracts.

A significant difference between the expression level of at least one biomarker of the biomarkers of Table 1 comprising at least apolipoprotein A-IV with a reference value is, according to the diagnostic method of the invention, indicative of glaucoma. More specifically, according to the diagnostic method of the invention, if the subject suffers from pseudoexfoliative syndrome, an increase in the expression level of at least one biomarker of the biomarkers of Table 1 comprising at least apolipoprotein A-IV compared to a reference value is indicative of PEXG and, if the subject does not suffer from pseudoexfoliative syndrome, an increase in the expression levels of at least one biomarker of the biomarkers of Table 1 except serum albumin and comprising at least apolipoprotein A-IV compared to a reference value, and/or a decrease in the expression level of serum albumin compared to a reference value is indicative of POAG.

The term "pseudoexfoliation syndrome" or "PEX", as used herein, refers to an age-related systemic microfibrillopathy that targets ocular tissues through the gradual deposition of fibrillary residue from the lens and iris pigment epithelium, mainly on the lens capsule, ciliary body, zonules, corneal endothelium and iris. The diagnosis of PEX is based in characteristic findings during the ophthalmic examination, like the presence of white fibrillary residue on the anterior lens capsule and pupillary margin, pupillary transillumination defects and pigmentation of the trabecular meshwork. According to the diagnostic method of the invention, a subject is considered to suffer from pseudoexfoliation syndrome if he/she is diagnosed from pseudoexfoliation syndrome and/or if he/she shows the symptoms of the syndrome.

The term "significant difference", as used herein, refers to any difference between the expression level of a biomarker and its reference value, which is higher than the experimental error of the measurement. The significant difference can be an increased or a decreased.

According to the diagnostic method of the invention, the expression level of a biomarker is considered "increased" when the level of said biomarker in a sample is higher than a reference value. The levels of a biomarker are considered to be higher than a reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference value.

Likewise, in the context of the diagnostic method of the invention, the level of a biomarker is considered "decreased" when the level of said biomarker in a sample is lower than a reference value. The levels of a biomarker are considered to be lower than a reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than a reference value. In a particular embodiment of the diagnostic method of the invention, an increase in the expression level of apolipoprotein A-IV and optionally of at least one biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, and apolipoprotein L1, compared to a reference value and/or an increase or a decrease in the expression level of serum albumin compared to a reference value is indicative of glaucoma. In a more particular embodiment, if the subject suffers from pseudoexfoliation syndrome, an increase in the expression level of apolipoprotein A-IV and optionally of at least one biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, apolipoprotein L1, and serum albumin compared to a reference value is indicative of PEXG. Further, in another more particular embodiment, if the subject does not suffer from pseudoexfoliation syndrome, an increase in the expression level apolipoprotein A-IV and optionally of at least one biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, and apolipoprotein L1 compared to a reference value and/or a decrease in the expression level of serum albumin compared to a reference value is indicative of POAG.

In a particular embodiment, the diagnostic method of the invention comprises the determination of the expression level of at least 1, at least 2, at least 3, at least 4, at least 5, at least 6, at least 7, at least 8, at least 9, at least 10, at least 11, at least 12, or at least 13 of the biomarkers of Table 1, wherein at least one of said biomarkers of Table 1 is apolipoprotein A-IV.

Thus, in a particular embodiment, the diagnostic method of the invention comprises the determination of the expression level of only one biomarker of the biomarkers listed in Table 1, wherein said biomarker is apolipoprotein A-IV.

In a particular embodiment, the diagnostic method of the invention comprises the determination of the expression level of apolipoprotein A-IV and the determination of at least 1, at least 2, at least 3, at least 4, at least 5, at least 6, at least 7, at least 8, at least 9, at least 10, at least 11, or at least 12, of the other biomarkers of Table 1.

In another particular embodiment, the diagnostic method of the invention comprises the determination of the expression level of all the 13 biomarkers of Table 1.

In a specific particular embodiment, the diagnostic method of the invention comprises determining the expression level of at least two biomarkers selected from the biomarkers listed in Table 1 wherein one of said two biomarkers of Table 1 is apolipoprotein A-IV . In another particular embodiment, the diagnostic method of the invention comprises determining the expression level of at least three biomarkers selected from the biomarkers listed in Table 1 wherein one of said three biomarkers of Table 1 is apolipoprotein A-IV. In a more specific particular embodiment, the diagnostic method of the invention comprises the determination of the biomarkers apolipoprotein A-IV, complement C3 and serotransferrin.

In another particular embodiment, the diagnostic method of the invention further comprises the determination of the expression level of an additional biomarker selected from the group consisting of biomarkers Ig gamma-2 chain C region, antithrombin-III, complement C4A, serum amyloid P-component and any combination thereof.

Thus, in a specific particular embodiment, the diagnostic method of the invention further comprises (i) the determination of the expression level of at least one of the biomarkers listed in Table 1 wherein at least one of said biomarkers of Table 1 is apolipoprotein A-IV, and (ii) the determination of the expression level of at least one additional biomarker selected from the group consisting of biomarkers Ig gamma-2 chain C region, antithrombin-III, complement C4A, serum amyloid P-component and any combination thereof.

Also, in a specific particular embodiment, the diagnostic method of the invention further comprises (i) the determination of the expression level of at least 2, at least 3, at least 4, at least 5, at least 6, at least 7, at least 8, at least 9, at least 10, at least 11, or at least 12 of the biomarkers listed in Table 1 wherein at least one of said biomarkers of Table 1 is apolipoprotein A-IV, and (ii) the determination of the expression level of at least one additional biomarker selected from the group consisting of biomarkers Ig gamma-2 chain C region, antithrombin-III, complement C4A, serum amyloid P-component and any combination thereof.

Alternatively, in a specific particular embodiment, the diagnostic method of the invention further comprises (i) the determination of the expression level of all the biomarkers listed in Table 1, and (ii) the determination of the expression level of at least one additional biomarker selected from the group consisting of biomarkers Ig gamma-2 chain C region, antithrombin-III, complement C4A, serum amyloid P-component and any combination thereof.

In a specific particular embodiment, the diagnostic method of the invention comprises (i) the determination of the expression level of at least two biomarkers of the biomarkers of Table 1, wherein one of these three biomarkers of Table 1 is apolipoprotein A-IV, and (ii) the determination of the expression level of at least one additional biomarker selected from the group consisting of Ig gamma-2 chain C region, antithrombin-III, complement C4A and serum amyloid P-component. In a more particular embodiment, said at least additional biomarker is complement C4A. Even more particular embodiments of the diagnostic method of the invention include the determination of the expression level of a combination or panel of biomarkers, wherein said combination of biomarkers comprises:
- biomarkers apolipoprotein A-IV, Ig gamma-2 chain C region, apolipoprotein L1 and complement C4A;
- biomarkers apolipoprotein A-IV, complement C3, complement C4A and serum amyloid P-component and alpha-1 antitrypsin,
- biomarkers apolipoprotein A-IV, complement C3, serotransferrin, apolipoprotein L1, Ig gamma-2 chain C region and complement C4A,
- biomarkers apolipoprotein A-IV, complement C4A, apolipoprotein L1, Ig gamma-2 chain C region, and complement C3;
- biomarkers apolipoprotein A-IV, complement C3, serotransferrin, and complement C4A;
- biomarkers apolipoprotein A-IV, complement C3, and serotransferrin; or
- biomarkers apolipoprotein A-IV, complement C3, serotransferrin, vitronectin, transthyretin, alpha-1 antitrypsin, fibulin-1, Apolipoprotein A1, ficolin-3, complement factor H, inter-alpha-trypsin inhibitor heavy chain H4, apolipoprotein L1, serum albumin, antithrombin-III, Ig gamma-2 chain C region, complement C4A and serum amyloid P-component.

The present disclosure also describes a diagnostic method including the determination of the expression level of a combination or panel of biomarkers, wherein said combination of biomarkers comprises
- biomarkers complement C3, serotransferrin, vitronectin, transthyretin, alpha-1 antitrypsin, fibulin-1, apolipoprotein A1, ficolin-3, complement factor H, inter-alpha-trypsin inhibitor heavy chain H4, apolipoprotein L1, serum albumin, antithrombin-III, Ig gamma-2 chain C region, and serum amyloid P-component;
- biomarkers serum albumin, antithrombin-III, Ig gamma-2 chain C region, complement C4A, and serum amyloid P-component;
- biomarkers serum albumin, antithrombin-III, Ig gamma-2 chain C region, complement C4A, serum amyloid P-component, inter-alpha-trypsin inhibitor heavy chain H4, and apolipoprotein L1;
- biomarkers Ig gamma-2 chain C region, complement C4A, serum amyloid P-component, and inter-alpha-trypsin inhibitor heavy chain H4;
- biomarkers antithrombin-III, Ig gamma-2 chain C region, complement C4A, serum amyloid P-component, and inter-alpha-trypsin inhibitor heavy chain H4;
- biomarkers complement C3, antithrombin-III, serotransferrin, and apolipoprotein L1; or
- biomarkers complement C3, antithrombin-III, serum amyloid P-component, and alpha-1 antitrypsin;

### Kits for the diagnosis of glaucoma

In another aspect, the invention relates to a kit, hereinafter referred to as "kit of the invention", comprising a reagent for determining the expression level of more than one biomarker selected from the biomarkers of Table 1 except apolipoprotein A-I, wherein at least one of said biomarkers of Table 1 is apolipoprotein A-IV wherein the reagent for determining the expression level is selected from the group consisting of: (a) an antibody, an aptamer, or a fragment thereof that specifically binds to the biomarker protein; and (b) a reagent suitable for determining the expression level of the biomarker by means of a multiple reaction monitoring (MRM) assay, said reagent being a isotope-labeled peptide obtainable by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin and endoproteinase GluC.

The term "kit", as used herein, refers to a product containing the different reagents necessary for carrying out the methods of the invention packed so as to allow their transport and storage. Materials suitable for packing the components of the kit include crystal, plastic (e.g., polyethylene, polypropylene, polycarbonate, etc.), bottles, vials, paper, or envelopes. The kit can also include instructions for use.

The biomarkers of Table 1 have been previously defined in relation with the diagnostic method of the invention.

The expression "reagent for determining the expression level of (a biomarker)", as used herein, refers to a compound (or set of compounds) that allows determining the level of expression of the particular biomarker. In a particular embodiment, the reagents for determining the expression level of a biomarker include compounds that bind specifically to the biomarker proteins selected from the group consisting of antibodies, aptamers or fragments thereof. More preferably, said compounds are antibodies or fragments thereof that specifically bind to the corresponding biomarkers.

In another particular embodiment, the reagents for determining the expression level of a biomarker includes reagents suitable for determining the expression levels of the biomarkers by means of a MRM assay. Said reagents are isotope-labeled peptides derived from the biomarker proteins whose level is going to be determined by MRM. Said isotope-labeled peptides are obtainable from the biomarker protein from which they are derived by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin or endoproteinase GluC. In another particular embodiment, the reagents for determining the expression level of a biomarker further include an enzyme capable of cleaving or hydrolyzing peptides or proteins into fragments in either specific or generic manner, preferably trypsin, papain, endoproteinase LysC, endoproteinase ArgC, staph aureus V8, chymotrypsin, Asp-N, Asn-C, pepsin, and endoproteinase GluC.

In another particular embodiment, the kit of the invention comprises quantum dots, more specifically, quantum dots coupled to compounds that bind specifically to the biomarker proteins; preferably quantum dots coupled to antibodies or fragments thereof that bind specifically to the biomarker proteins.

In another embodiment, the kit of the invention comprises a reagent for determining the expression level of at least 2, at least 3, at least 4, at least 5, at least 6, at least 7, at least 8, at least 9, at least 10, at least 11 or at least 12, or even all (13), of the biomarkers listed in Table 1 except apolipoprotein A-I, wherein at least one of said biomarkers of Table 1 is apolipoprotein A-IV. In a specific embodiment, the kit of the invention comprises a reagent for determining the expression level of (i) a first biomarker, wherein said first biomarker is apolipoprotein A-IV, and (ii) a reagent for determining the expression level of at least 1, at least 2, at least 3, at least 4, at least 5, at least 6, at least 7, at least 8, at least 9, at least 10, at least 11 or at least 12, of the other biomarkers listed in Table except apolipoprotein A-I. In a more specific embodiment, said first biomarker is an antibody, or a fragment thereof, that specifically binds to apolipoprotein A-IV.

In another particular embodiment, the kit of the invention comprises a reagent for determining the expression level of biomarker apolipoprotein A-IV and at least a reagent for determining the expression level of at least 1, at least 2, at least 3, at least 4, at least 5, at least 6, at least 7, at least 8, at least 9, at least 10, at least 11 or at least 12, of the other biomarkers listed in Table 1 except apolipoprotein A-I.

In another particular embodiment, the kit of the invention comprises reagents for determining the expression level of all the 13 biomarkers of Table 1 except apolipoprotein A-I.

In a specific particular embodiment, the kit of the invention comprises reagents for determining the expression level of at least three biomarkers selected from the biomarkers listed in Table 1 except apolipoprotein A-I, preferably antibodies, or fragments thereof, that specifically bind to said at least three biomarkers selected from the biomarkers of Table 1 except apolipoprotein A-I, wherein at least one of said three biomarkers of Table 1 is apolipoprotein A-IV. In a more specific particular embodiment, the kit of the invention comprises a reagent
for determining the expression level of apolipoprotein A-IV, a reagent for determining the expression level of complement C3 and a reagent for determining the expression level of serotransferrin; preferably, said reagents are antibodies, or fragments thereof, that specifically bind to said biomarkers.

In another particular embodiment, the kit of the invention further comprises a reagent for determining the expression level of at least one additional biomarker, wherein said additional biomarker is selected from the group consisting of biomarkers Ig gamma-2 chain C region, antithrombin-III, complement C4A, serum amyloid P-component and any combination thereof.

Thus, in a specific particular embodiment, the kit of the invention comprises (i) reagents for determining at least two of the biomarkers listed in Table 1 except apolipoprotein A-I, wherein at least one of said biomarkers of Table 1 is apolipoprotein A-IV, and (ii) reagents for determining at least one additional biomarker selected from the group consisting of biomarkers Ig gamma-2 chain C region, antithrombin-III, complement C4A, serum amyloid P-component and any combination thereof.

Also, in a specific particular embodiment, the kit of the invention further comprises (i) reagents for determining at least 2, at least 3, at least 4, at least 5, at least 6, at least 7, at least 8, at least 9, at least 10, at least 11, or at least 12 of the biomarkers listed in Table 1 except apolipoprotein A-I, wherein at least one of said biomarkers of Table 1 is apolipoprotein A-IV, and (ii) reagents for determining at least one additional biomarker selected from the group consisting of biomarkers Ig gamma-2 chain C region,, antithrombin-III, complement C4A, serum amyloid P-component and any combination thereof. Alternatively, in a specific particular embodiment, the kit of the invention further comprises (i) reagents for determining all the biomarkers listed in Table 1 except apolipoprotein A-I, and (ii) reagents for determining at least one additional biomarker selected from the group consisting of biomarkers serum Ig gamma-2 chain C region, antithrombin-III, complement C4A, serum amyloid P-component and any combination thereof.

In another particular embodiment, the kit of the invention comprises reagents for determining the expression level of (i) at least two biormarkers of the biomarkers listed in Table 1 except apolipoprotein A-I, wherein one of said two biomarkers of Table 1 is apolipoprotein A-IV, and (ii) at least one additional biomarker selected from the group of biomarkers consisting of serum Ig gamma-2 chain C region, antithrombin-III, complement C4A, serum amyloid P-component and combinations thereof.

In another particular embodiment, the kit of the invention comprises reagents for determining the expression level of a combination of biomarkers, wherein said combination of biomarkers comprises:
- biomarkers apolipoprotein A-IV, Ig gamma-2 chain C region, apolipoprotein L1 and complement C4A;
- biomarkers apolipoprotein A-IV, complement C3, complement C4A and serum amyloid P-component and alpha-1 antitrypsin;
- biomarkers apolipoprotein A-IV, complement C3, serotransferrin, apolipoprotein L1, Ig gamma-2 chain C region and complement C4A;
- biomarkers apolipoprotein A-IV, complement C4A, apolipoprotein L1, Ig gamma-2 chain C region, and complement C3;
- biomarkers apolipoprotein A-IV, complement C3, serotransferrin, and complement C4A;
- biomarkers apolipoprotein A-IV, complement C3, and serotransferrin or
- biomarkers apolipoprotein A-IV, complement C3, serotransferrin, vitronectin, transthyretin, alpha-1 antitrypsin, fibulin-1, ficolin-3, complement factor H, inter-alpha-trypsin inhibitor heavy chain H4, apolipoprotein L1, serum albumin, antithrombin-III, Ig gamma-2 chain C region, complement C4A and serum amyloid P-component.

The present disclosure further describes a diagnostic method including the determination of the expression level of a combination or panel of biomarkers, wherein said combination of biomarkers comprises
- biomarkers complement C3, serotransferrin, vitronectin, transthyretin, alpha-1 antitrypsin, fibulin-1, apolipoprotein A1, ficolin-3, complement factor H, inter-alpha-trypsin inhibitor heavy chain H4, apolipoprotein L1, serum albumin, antithrombin-III, Ig gamma-2 chain C region, and serum amyloid P-component;
- biomarkers serum albumin, antithrombin-III, Ig gamma-2 chain C region, complement C4A, and serum amyloid P-component;
- biomarkers serum albumin, antithrombin-III, Ig gamma-2 chain C region, complement C4A, serum amyloid P-component, inter-alpha-trypsin inhibitor heavy chain H4, and apolipoprotein L1;
- biomarkers Ig gamma-2 chain C region, complement C4A, serum amyloid P-component, and inter-alpha-trypsin inhibitor heavy chain H4;
- biomarkers antithrombin-III, Ig gamma-2 chain C region, complement C4A, serum amyloid P-component, and inter-alpha-trypsin inhibitor heavy chain H4;
- biomarkers complement C3, antithrombin-III, serotransferrin, and apolipoprotein L1; or
- biomarkers complement C3, antithrombin-III, serum amyloid P-component, and alpha-1 antitrypsin.

In a particular embodiment of the kit of the invention, the reagents for determining the expression level of the biomarkers are, preferably, antibodies, or fragments thereof, that specifically bind to said biomarkers.

In all the embodiments of the kits previously mentioned, said reagents for determining the expression level of the additional biomarkers are, preferably, antibodies, or fragments thereof, that specifically bind to said additional biomarker.

The antibodies, or fragments thereof, of the kit of the invention can be used according to techniques known in art for determining protein expression levels such as, for example, flow cytometry, Western blot, ELISA, RIA, competitive EIA, DAS-ELISA, techniques based on the use of biochips, protein microarrays, assays of colloidal precipitation in reactive strips or multiplex assays based on antibody-linked quantum dots. In a particular embodiment, the kit of the invention can be used to determine the expression level of the biomarkers by means of multiple reaction monitoring (MRM) in a quantitative tandem mass spectrometry approach.

The antibodies can be fixed to a solid support such as a membrane, a plastic or a glass, optionally treated to facilitate the fixation of said antibodies to the support. Said solid support may comprise, at least, a set of antibodies which specifically recognize the first biomarker panel and at least one biomarker of the second biomarker panel and which can be used for detecting the levels of expression of said biomarker.

Additionally, the kits of the invention may comprise reagents for detecting control proteins. The availability of said additional reagents allows normalizing the measurements performed in different samples, for example, the sample to be analyzed and the control sample, to rule out that the differences in the expression of the biomarkers are due to a different quantity of total protein amount in the sample more than the real differences in the relative levels of expression. The control proteins useful for the kits of the present invention are proteins whose expression levels are not affected by glaucoma, for example serum albumin.

In a preferred embodiment, the reagents for assaying the levels of the different biomarkers comprise at least 10%, at least 20%, at least 30%, at least 40%, at least 50%, at least 60%, at least 70%, at least 80%, at least 90% or at least 100% of the total amount of reagents for assaying biomarkers forming the kit. Thus, in the particular case of kits comprising reagents for assaying the levels of apolipoprotein A-IV, complement C3 and serotransferrin, the reagents specific for said biomarkers, e.g., antibodies, or functional fragments thereof (i.e., fragments of antibodies having the ability to recognize the corresponding antigen), which bind specifically to apolipoprotein A-IV, complement C3 and serotransferrin, respectively) comprise at least 10%, at least 20%, at least 30%, at least 40%, at least 50%, at least 60%, at least 70%, at least 80%, at least 90%, or at least 100% of the total amount of antibodies, or functional fragments thereof, present in the kit. Further, in the particular case of kits comprising reagents for assaying the levels of apolipoprotein A-IV, complement C4A, Ig gamma-2 chain C region and apolipoprotein L1, the reagents specific for said biomarkers, e.g., antibodies, or functional fragments thereof which bind specifically to apolipoprotein A-IV, complement C4A, Ig gamma-2 chain C region and apolipoprotein L1, comprise at least 10%, at least 20%, at least 30%, at least 40%, at least 50%, at least 60%, at least 70%, at least 80%, at least 90%, or at least 100% of the total amount of antibodies, or functional fragments thereof, present in the kit. Further, in the particular case of kits comprising reagents for assaying the levels of apolipoprotein A-IV, complement C4A, complement C3, serum amyloid P-component and alpha-1 antitrypsin, the reagents specific for said biomarkers, e.g., antibodies, or functional fragments thereof which bind specifically to apolipoprotein A-IV, complement C4A, complement C3, serum amyloid P-component and alpha-1 antitrypsin, comprise at least 10%, at least 20%, at least 30%, at least 40%, at least 50%, at least 60%, at least 70%, at least 80%, at least 90%, or at least 100% of the total amount of antibodies, or functional fragments thereof, present in the kit. Further, in the particular case of kits comprising reagents for assaying the levels of apolipoprotein A-IV, complement C4A, complement C3, serotransferrin, apolipoprotein L1 and Ig gamma-2 chain C region, the reagents specific for said biomarkers, e.g., antibodies, or functional fragments thereof which bind specifically to apolipoprotein A-IV, complement C4A, complement C3, serotransferrin, apolipoprotein L1 and Ig gamma-2 chain C region, comprise at least 10%, at least 20%, at least 30%, at least 40%, at least 50%, at least 60%, at least 70%, at least 80%, at least 90%, or at least 100% of the total amount of antibodies, or functional fragments thereof, present in the kit. Further, in the particular case of kits comprising reagents for assaying the levels of apolipoprotein A-IV, complement C4A and all the biomarkers from Table 1, the reagents specific for said biomarkers, e.g., antibodies, or functional fragments thereof which bind specifically to apolipoprotein A-IV, complement C4A and all the biomarkers from Table 1, comprise at least 10%, at least 20%, at least 30%, at least 40%, at least 50%, at least 60%, at least 70%, at least 80%, at least 90%, or at least 100% of the total amount of antibodies, or functional fragments thereof, present in the kit. Further, in the particular case of kits comprising reagents for assaying the levels of any of the panel or combination of biomarkers mentioned above, the reagents specific for said biomarkers, e.g., antibodies, or functional fragments thereof which bind specifically to the biomarkers of said panel or combination of biomarkers, comprise at least 10%, at least 20%, at least 30%, at least 40%, at least 50%, at least 60%, at least 70%, at least 80%, at least 90%, or at least 100% of the total amount of antibodies, or functional fragments thereof, present in the kit.

The kit of the invention is particularly useful for diagnosis of a glaucoma selected from POAG and PEGX. Thus, in another aspect, the invention relates to the use of a kit comprising a reagent for determining the expression level of at least one biomarker selected from the biomarkers listed in Table 1 wherein at least one of said biomarkers of Table 1 is apolipoprotein A-IV for diagnosis of a glaucoma selected from primary open angle glaucoma and pseudoexfoliation glaucoma, wherein the reagent for determining the expression level is selected from the group consisting of: (a) an antibody, an aptamer, or a fragment thereof that specifically binds to the biomarker protein; and (b) a reagent suitable for determining the expression level of the biomarker by means of a multiple reaction monitoring (MRM) assay, said reagent being a isotope-labeled peptide obtainable by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin and endoproteinase GluC. In a particular embodiment, said reagent is an antibody, or fragment thereof, which specifically binds to said biomarker.

In other words, the invention relates to a kit comprising a reagent for determining the expression level of at least one biomarker selected from the biomarkers listed in Table 1, wherein at least one of said biomarkers of Table 1 is apolipoprotein A-IV for use in the diagnosis of a glaucoma selected from POAG and PEXG, wherein the reagent for determining the expression level is selected from the group consisting of: (a) an antibody, an aptamer, or a fragment thereof that specifically binds to the biomarker protein; and (b) a reagent suitable for determining the expression level of the biomarker by means of a multiple reaction monitoring (MRM) assay, said reagent being a isotope-labeled peptide obtainable by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin and endoproteinase GluC. In a particular embodiment, said reagent is an antibody, or fragment thereof, which specifically binds to said biomarker.

The following example is included for illustrative, non-limitative, purposes.

### EXAMPLE 1

### Differential Proteomics-based biomarkers in serum of patients with primary open-angle glaucoma and pseudoexfoliation glaucoma

### 1. METHODS

### Study design

The workflow of the present study consisted of two phases:
- Phase 1 (Discovery) including study population, sample collection, serum sample preparation, 2D-DIGE analysis, image acquisition and data analysis, and protein identification; and
- Phase 2 (Validation) including biomarker validation by ELISA analysis and multivariate predictive models generation.

Each step is described in detail separately.

### Phase 1 - Discovery; this phase included:

i) Study population. POAG, PEXG, and control cases were screened and selected through the Instituto Oftalmológico Fernández-Vega (IOFV) and classified according to their age and gender;
ii) Sample colletcion. Blood samples were drawn from each participant, and the sera were separated from clotting factors and blood cells by centrifugation;
iii) Serum sample preparation. Serum samples were subjected to protein equalization using ProteoMiner to reduce the dynamic range of the highly abundant proteins;
iv) 2D-DIGE. Labeling of serum proteins was carried out *in vitro* with cyanine dyes (CyDye) (Cy2, Cy3, Cy5), and the proteins were separated by 2D-DIGE;
v) Image Acquisition and Data analysis. Gels were scanned and image analysis was performed with Progenesis SameSpots Software; and
vi) Protein Identification. Protein spots were isolated and their identification carried out by MALDI TOF/TOF and nLC-MS/MS.

### Phase 2 - Validation; this phase included:

vii) Biomarker Validation. Quantitative determination of the discriminatory nature of the proteins identified as potential biomarkers was performed through individually-available enzyme-linked immunosorbent assay (ELISA) analysis. Assays were carried out on some of the same samples included in phase one of this study, as well as in new POAG, PEXG, and control cases; and
viii) Univariate and multivariate Predictive Models. Once the discriminatory nature of the biomarkers was quantified, their concentrations were used to construct multivariate predictive models capable of segregating POAG, PEXG, and control cases with the best discrimination power.

### Study population

The study adheres to the tenets of the Declaration of Helsinki, and full ethical approval was obtained from the Clinical Research Ethics Committee at the Hospital Universitario Central de Asturias (Oviedo, Spain). All patients included in this study were recruited, signed an informed consent, and had complete ophthalmologic examinations at the IOFV. A total of 202 patients were enrolled in this study. In the discovery phase, 53 POAG, 45 PEXG and 51 control patients were included (n=149, see Table 2).

**Table 2**

| **Demographic details of control, POAG and PEXG groups involved in the discovery phase** | | | |
|---|---|---|---|
| | **Pathological Status** | | |
| | **Control** | **POAG** | **PEXG** |
| | **(n = 51)** | **(n = 53)** | **(n = 45)** |
| Age, y | | | |
| Mean ± SD^{a} | 64.93 ± 11.34 | 67.46 ± 11.27 | 73.11 ± 8.43 |
| Range | 43 - 87 | 35 - 89 | 55 - 92 |
| *p*-value, vs. control^{b} | - | 0.3054 | 0.0001 |
| *p*-value, vs POAG^{b} | - | - | 0.0049 |

| Sex | | | |
|---|---|---|---|
| Men, n (Mean age ± SD)^{a} | 24 (64.67 ± 9.33) | 29 (65.00 ± 11.17) | 22 (73.09 ± 8.16) |
| Women, n (Mean age ± SD)^{a} | 27 (65.19 ± 13.05) | 24 (69.92 ± 11.03) | 23 (73.13 ± 8.87) |

| | | | |
|---|---|---|---|
| ^{a}Data are shown as average ± standard deviation ^{b}*p*-values are calculated between each pair of groups using the Unpaired t test with Welch correction. | | | |

In the validation phase, 30 POAG, 29 PEXG and 29 control patients were included (n=88), of which 10 POAG, 15 PEXG and 10 control were also incorporated in discovery phase (see Table 3).

**Table 3**

| **Demographic details of control, POAG and PEXG groups used for ELISA assays** | | | |
|---|---|---|---|
| | **Pathological Status** | | |
| | **Control** | **POAG** | **PEXG** |
| | **(n = 29)** | **(n = 30)** | **(n =29)** |
| Age, y | | | |
| Mean ± SD^{a} | 64.45 ± 12.39 | 69.30 ± 9.00 | 73.48 ± 6.74 |
| Range | 48 - 92 | 48 - 84 | 56 - 92 |
| *p*-value, vs. control^{b} | - | 0.0922 | 0.0013 |
| *p*-value, vs POAG^{b} | - | - | 0.0479 |

| Sex | | | |
|---|---|---|---|
| Men, n (Mean age ± SD)^{a} | 14 (64.07 ± 13.90) | 16 (69.06 ± 7.86) | 16 (74.69 ± 7.19) |
| Women, n (Mean age ± SD)^{a} | 15 (64.80 ± 11.28) | 14 (69.57 ± 10.45) | 13 (72.00 ± 6.10) |

| | | | |
|---|---|---|---|
| ^{a}Data are shown as average ± standard deviation ^{b}*p*-values are calculated between each pair of groups using the Unpaired t test with Welch correction. | | | |

The diagnostic criteria for glaucoma, both primary open-angle and pseudoexfoliation, was the presence of characteristic optic-disc damage (e.g., vertical cup-to-disc ratio >0.3, thin or notched neuroretinal rim, or disc hemorrhage) with the corresponding characteristic changes in the visual field and the presence of open anterior chamber angle (Shaffer grade III or IV). POAG patients presented without secondary causes of optic neuropathy, while the subjects with PEXG exhibited characteristic exfoliative material on the anterior lens surface and/or iris during slit-lamp examination, in one or both eyes. Control subjects were selected from patients undergoing cataract surgery. Of note, most glaucoma patients also had cataracts (90.4% and 94.9% of POAG and PEXG subjects, respectively). No subjects involved in this study presented with other relevant ocular pathologies such as clinically detectable inflammation, infection, retinopathies, or maculopathies.

### Sample collection

Blood was collected in 5 mL Z Serum Sep Clot Activator tubes coated with microscopic silica particles, which activates the coagulation process (Vacuette, Madrid, Spain). Tubes were centrifuged at 1,800 g for 18 min at 4°C, and the supernatant (serum) was stored at -80°C until use. Blood from glaucoma and control groups was collected and processed in an identical manner.

### Serum sample preparation and quantification

Since the protein concentration in sera samples is very high (60-80 mg/mL), and approximately 95% of all the protein content is represented by fourteen proteins (a high dynamic range), a preliminary step before 2D-DIGE known as "equalization" was introduced. Equalization involved the ProteoMiner™ Small-Capacity Kit (BioRad Laboratories, Hercules, CA, USA), which increases the representation (i.e., concentration) of the proteins within the medium and low dynamic ranges. Thus, instead of depleting the highly abundant proteins, the ProteoMiner approach reduces the dynamic range by equalizing their concentrations with those of the more scarce proteins. The ProteoMiner kit is based on technology that uses a combinatorial ligand library of 10⁹ - 10¹² uniquely-degenerated hexapeptide ligands, each of which is bound to a resin bead. Each protein in the serum sample is expected to have the same probability to bind to its unique hexapeptide ligand/resin bead present in the ProteoMiner and therefore retained. Thus, each ligand may bind a unique protein or protein complex. Since the number of hexapeptide ligands represented in the ProteoMiner kit for each protein is the same, the most abundant proteins will rapidly saturate their unique hexapeptide ligands and the excess of unbound proteins will be eliminated. Under conditions of saturation (i.e., > 10 mg protein per sample/chromatographic separation), and following the manufacturer's protocol of the ProteoMiner kit, the concentrations of abundant proteins in serum samples are reduced while those of less concentrated proteins are enriched.

Upon equalization, serum proteins were precipitated to remove lipids with CleanUp kit (GE Healthcare, Sunnyvale, CA, USA), solubilized in 30 µl of DIGE (difference gel electrophoresis) compatible focusing solution (30 mM Tris-HCl, pH 8.5, 2M ThioUrea, 7M Urea and 4% CHAPS (3-[(3-cholamidopropyl)dimethylammonio]-1-propanesulfonate)) and stored at -80°C until use.

The protein concentration in each serum sample was determined before its fractionation through ProteoMiner, according to the Bradford method, and upon equalization, by the EZQ Protein Quantification Kit (Invitrogen Dynal AS, Oslo, Norway).

### 2D - DIGE for biomarker discovery

In the present study 2D-DIGE was used to identify changes in the concentrations of proteins between equalized serum samples from POAG, PEXG, and control patients. Three different samples were run together in one gel. Proteins in each sample were labeled with distinct fluorescent dyes: Cy2, Cy3 and Cy5. The Cy2 labeled proteins corresponded to an internal standard (see below), which was included in each 2D-DIGE gel run. The other two samples in each gel corresponded to equalized serum from POAG, PEXG or control patients, and they were labeled with Cy3 or Cy5. This approach reduced by approximately half the total number of 2D gels needed when compared to conventional two-dimensional gel electrophoresis method. A total of 75 gels (i.e., 2D-DIGE) were run and analyzed.

The internal standard was generated by mixing equimolar amounts of each of the equalized sera samples included in this study (i.e., 149). The pool of proteins in this internal standard was minimally labeled with Cy2 using CyDye, according to the manufacturer's protocol (GE Healthcare, Sunnyvale, CA, USA). The proteins in the other two samples (equalized sera) were labeled with either Cy3 or Cy5 at a pH of 8-9. The internal standard was used to normalize for differences in protein loading, gel-to-gel variations, as well as aid in the alignment of scanned images. Under these conditions, the abundance of each protein in every sample could be normalized relative to the internal standard. Because the spectral properties of Cy2, Cy3, and Cy5 are not identical, we introduced a *Compensation Flip Step,* so that Cy3- and Cy5-labeled proteins from samples of each group under study (i.e., Cy3-POAG and Cy5-PEXG; Cy3-PEGX and Cy5-POAG; Cy3-POAG and Cy5-Control; Cy3-Control and Cy5-POAG; Cy3-PEGX and Cy5-Control; Cy3-Control and Cy5-PEXG) would be represented in approximately equal numbers.

Labeling of the proteins was performed by the addition of 400 pmol of the required CyDye per 50 µg of protein in 1 µl of anhydrous N,N-dimethylformamide. The labeling reaction was carried out during 30 min on ice and in the dark, and 1 µl of 10 mM lysine (Sigma Aldrich, St. Louis, MO, USA) was added for 10 min to terminate the reaction. Before 2D gel separation, the three samples labeled with Cy2, Cy3 and Cy5 were pooled and mixed with rehydration buffer [30 mM Tris-HCl, pH 8.5, 2M ThioUrea, 7M Urea and 4% CHAPS, 20 mM dithiothreitol (DTT) and 0.25 % pH 4-7 carrier ampholyte (Bio-lyte; Bio-Rad, Hercules, CA, USA)]. The samples were subsequently applied to immobilized pH gradient strips (24 cm, pH 4-7) and focused in a Protean Isoelectrofocusing Cell (BioRad, Malvern, Pennsylvania, USA) following instructions by the supplier. The focused strips were equilibrated for 15 min with buffer 1 (6 M urea, 0.375 M Tris-HCl, 2% SDS, 20% glycerol) supplemented with 2% (w/v) DTT, then subsequently incubated another 15 min with buffer 1 supplemented with 2.5% (w/v) iodoacetamide.

The second-dimension separation was carried out by sodium dodecyl sulfate-polyacrylamide gel electrophoresis (SDS-PAGE) in large 12.5% polyacrylamide gels using the DALT-SIX electrophoresis system (GE Healthcare, Sunnyvale, CA, USA) according to standard protocols. All electrophoresis procedures (labeling, 1^{st}- and 2^{nd}-dimensional separations) were performed in the dark.

### Image acquisition and data analysis

Images of Cy2-, Cy3- and Cy5- labeled proteins were taken after protein separation in each gel (i.e., 75 gels 2D-DIGE) upon excitation at the wavelengths of 488, 532, and 633 nm while capturing the emission signal at 530, 605 and 695 nm, respectively, using a VersaDoc Imaging System (BioRad, Malvern, Pennsylvania, USA). Gels were scanned utilizing 180 seconds of light exposure for each of the channels.

Image analysis of Cy2-, Cy3-, and Cy5- labeled proteins in each gel was performed with the *Progenesis SameSpots* Software, version 4.0 (NonLinear Dynamics Limited, Newcastle, UK). Variation in abundance of proteins (spots) between groups (i.e., POAG, PEXG, and controls) was determined by the Significance Analysis of Microarrays (SAM) method (Quigley HA, 2006, J Ophthalmol 90(3): 262-7), using normalized "spot" volumes (obtained from Progenesis SameSpots Software) with a cutoff False-Discovery-Rate value of 0.01.

### Mass Spectrometry Analysis

In order to identify protein spots by mass spectrometry (MS), inventors first had to isolate them. Separation was performed with a 12.5% SDS preparative gel, onto which 400 µg of protein from a pooled sample, which included an equimolar amount of all samples analyzed in the present study, was loaded and stained with SYPRO Ruby (BioRad, Malvern, Pennsylvania, USA). Protein spots with significant statistical power were robotically excised with an Ettan Spot Picker (GE Healthcare) from the preparative gel and subjected to in-gel trypsin digestion according to Shevchenko (Quigley HA, 2006, J Ophthalmol 90(3): 262-7), with the following minor modifications. The gel pieces were reduced with 30 µl of 10 mM DTT at 56°C for 20 min, followed by alkylation in 30 µl of 50 mM iodoacetamide at room temperature for 20 min in darkness. Next, spots were swollen in an ice bath with a digestion buffer containing 50 mM ammonium bicarbonate and 12.5 ng/µl of trypsin (Roche Diagnostics, recombinant, proteomics grade trypsin, Penzberg, Germany). After 30 min, the supernatant was removed and discarded, 20 µl of 50 mM ammonium bicarbonate was added to the gel piece, and the digestion allowed proceeding at 37 °C overnight. After trypsinization, the supernatant was transferred to an eppendorf and acidified to 0.1% trifluoroacetic acid.

### Protein identification MALDI-TOF/TOF

MALDI-MS analysis was performed with a MALDI-LIFT-TOF AUTOFLEX III SmartBeam (Bruker Daltonics). Each digested sample was loaded (1 µl) onto a target (Bruker 384 ground steel) with 1 µl of α-cyano-4-hydroxycinnamic acid. Data-dependent MS acquisitions were performed with a charge state of 1 over a survey mass-to-charge ratio range of 500-4000. Ionization was performed with a solid-state laser with wavelenght 360 nm and frequency 200 Hz. Laser intensity energies were varied depending on the analysis required. For MS, 30-50% of intensity was used, while around 90% for MS/MS. Resolution was always over 7500 along all mass-window ranges for MS analysis. Data acquisition was performed manually. Routinely 1400 scans were collected for Peptide Mass Fingerprint (PMF), whereas the most intense peaks were selected for MS/MS (400 scans for parent selection, and 1600 scans for fragments). Obtained spectra were processed using Flex analysis 3.0 and Biotools 3.2 (Bruker Daltonics). A database search was performed using MASCOT 2.2 (Matrixscience, London, UK) against UniProtKB/Swiss-Prot database (version SwissProt 2011_08; 531,473 sequences entries). The following parameters were adopted for protein identification: carbamidomethylation of cysteines as fixed modification, oxidation of methionines as variable modification, 50 ppm of peptide mass tolerance, 0.7 Da fragment mass tolerance, and up to two missed cleavage points. Calibration was performed externally, with Pepmix (Bruker Daltonics), and internally with trypsin peptides, when possible.

### Protein identification by nLC-MS/MS

Tryptic peptides were preconcentrated and desalted using a Symmetry C18 precolumn (180 µm x 20mm, 5 µm particle size, Waters) followed by elution on a BEH C130 column (75 µm × 200 mm, 1.7 µm particle size, Waters) using two solvents, A (0.1% formic acid) and B (0.1% formic acid in acetonitrile), with a linear gradient of solvent B from 3% to 50% in 50 min. All high-performance liquid chromatography runs were performed using a Waters nanoACQUITY nHPLC system (Waters) under a constant flow rate of 300 nL/min. The eluting peptides were scanned and fragmented with a LTQ Orbitrap XL mass spectrometer (Thermo Fisher Scientific) equipped with a Proxeon nano-electrospray source. An electrospray voltage of 1.6 kV and a capillary voltage of 40 V at 280°C were used. Survey scans ranging from 400 to 2000 mass-to-charge ratio were performed in the Orbitrap analyzer (30000 FWHM). MS/MS fragmentation and measurement of the six most intense ions were performed using collision-induced-dissociation in the LTQ linear ion trap. Normalized collision energy was set to 35%. Searches were performed using Proteome Discoverer 1.3 software (Thermo Fisher Scientific) and Mascot search engine. A tolerance of 5 ppm was allowed for the precursor search, whereas fragments were analyzed with a tolerance of 0.5 Da. Cystein carbamidomethylation was considered as fixed modification and methionine oxidation was considered as variable modification. Two missed cleavages were allowed for tryptic digestion. Spectra were searched against all entries of the UniProtKB/Swiss-Prot database, and proteins with at least two identified peptides (*p*<0.05) were considered significant hits.

### Validation of candidate biomarkers and Data Analysis

Validation of the protein markers in serum from POAG, PEXG, and control patients was performed on a total of 88 samples, of which 35 were included in phase 1 (10 POAG, 15 PEXG and 10 control) and 53 came from newly diagnostic cases (20 POAG, 14 PEXG and 19 control) (see Table 3). Sandwich-type ELISA assays were carried out on the 17 top-most-significant proteins from a total of 35 candidates identified by 2D-DIGE and MS analysis. ELISA kits were purchased from USCN Life Science Inc. (Wuhan, China) for the following proteins: CFH, C3, FBLN1, ALB, ITIH4, TF, APOA1, VTN, APOA4, IGHG2, APCS, APOL1, FCN3, SERPINA1, TTR. An ELISA kit for C4A was purchased from BD Biosciences (San Jose, CA, USA). An ELISA kit for SERPINC1 was obtained from AssayPro LLC (St. Charles, MO, USA). All ELISA assays were performed following the instructions described by the manufacturers.

The concentrations of each of the 17 proteins in the distinct sample groups (i.e., POAG, PEXG, and control) were determined by ELISA, and expressed with respect to serum volume. Feature subset subtraction was performed by stepwise discriminant analysis using the 'candisc' package in R software (http://www.r-project.org/), and a number of statistical tools based on *Machine Learning* approaches were applied in order to assess which method provided the best accuracy for the correct clinical classification of samples based on the selected panel of protein markers. These tools included Receiver Operating Characteristic (ROC) curve analyses for each of the markers, Naive Bayes (NB), k-Nearest Neighbor (kNN), Random Forest (RF), Classification Trees, and an ensemble of Support Vector Machine and Naive Bayes algorithms (AdaBoost.M1). Each algorithm was trained by the biomarker panel. To assess learner performance, a 5-fold random sampling was carried out with 75% of the samples as a training set and the remaining 25% as a test set. Statistical analyses were carried out using Orange Canvas software v2.6 (http://orange.biolab.si).

### 2. RESULTS

### Subjects characteristics

In Phase 1 (discovery), the participants' mean age (±SD) in the POAG (n=53) and control (n=51) groups were comparable (67.46±11.27 years for POAG patients compared with 64.93±11.34 years for controls, respectively; *p>0.05*)*.* The mean age in the PEXG group (n=45; 73.11±8.43 years) was slightly higher than that of the POAG (*p*<*0.05*) and control (*p*<*0.001*) groups. By gender, the number of men and women in each group as well as their mean ages were similar (Table 2).

In Phase 2 (validation), the participants' mean age (±standard deviation (SD)) of POAG (n=30) and control (n=29) patients were comparable (69.30±9.00 years for POAG, compared with 64.45±12.39 years for control, respectively; *p*>*0.05*). The mean age the PEXG group (n=29; 73.48±6.74 years) was slightly higher than that of the POAG (*p*<*0.05*) and control (*p*<*0*.*01*) groups. By gender, the number of men and women in each group as well as their mean ages were similar (Table 3).

### 2D-DIGE analysis of serum proteome

A total of 75 gels (2D-DIGE) were needed to analyze 149 equalized serum samples (53 POAG, 45 PEXG, 51 Control) as described in Materials and Methods. Proteins were separated in the first dimension along an isoelectric point (pI) range of 4 to 7, and in the second dimension between molecular masses of 10 kDa and 150 kDa. To avoid labeling bias of proteins in each sample with Cy3 or Cy5, a *Dye-Flip* design was applied. Following the successfully warping the gels and importing their images into the Progenesis SameSpots program, an equalized serum protein map was obtained for every sample. A total of 823 protein spots were detected using the VersaDoc Imaging System Software. After normalization of the spot volumes (area x intensity), the data was expressed in matrix notation and filtered by means of a False-Discovery-Rate cutoff value of 0.01 (*q*-value<0.01), which resulted in 214 significant spots. Protein spots that displayed significant fold-change alterations in expression between POAG or PEXG and control cases were ranked from highest to lowest discriminating power, based upon their ReliefF, Information gain and Gain Ratio values. A total of 149 protein spots displaying significant changes in abundance were selected for identification purposes by specifying a threshold of Gain ratio≥0.05, and subsequently classified according to their Spot ID, RefiefF, Information Gain, and Gain Ratio values.

### Identification of most differentially expressed proteins

The above referenced 149 protein spots were excised from a preparative 2D-PAGE gel, and they were subjected to in-gel trypsin digestion and MALDI-TOF/TOF or nLC-MS/MS analysis for identification, as described in Material and Methods. The protein identity of 118 spots out of the total 149 protein spots was established, resulting in the identification of 35 distinct proteins. Fourteen of these proteins were present in single spots, while the rest were found in two or more spots. These latter spots could be the result of post-translational modifications including proteolytic cleavages of specific proteins. Ten protein spots remain unidentified, most likely due to their low concentration. Twenty-one protein spots were identified as human keratins, which are non-seric proteins, and they were not taken into consideration or included for further analysis because they most likely represent contaminants from gel handling. Table 4 shows the list of the 35 distinct proteins identified by 2D-DIGE differentially expressed between the three sample groups and ranked by statistical significance from highest to lowest.

**Table 4**

| **List of the 35 differentially expressed proteins in patients suffering glaucoma compared to controls, sorted in descending order of significance** | | | |
|---|---|---|---|
| **Discrimination ranking** | **UniProt Accession number** | **Protein name** | **Gene name** |
| 1 | P08603 | Complement factor H | CFH |
| 2 | P23142 | Fibulin-1 | FBLN1 |
| 3 | P01024 | Complement C3 | C3 |
| 4 | Q14624 | Inter-alpha-trypsin inhibitor heavy chain H4 | ITIH4 |
| 5 | P01008 | Antithrombin-III | SERPINC1 |
| 6 | P02787 | Serotransferrin | TF |
| 7 | P02768 | Serum albumin | ALB |
| 8 | P04004 | Vitronectin | VTN |
| 9 | P01859 | Ig gamma-2 chain C region | IGHG2 |
| 10 | P02743 | Serum amyloid P-component | APCS |
| 11 | P01009 | Alpha-1 antitrypsin | SERPINA1 |
| 12 | P06727 | Apolipoprotein A-IV | APOA4 |
| 13 | O14791 | Apolipoprotein L1 | APOL1 |
| 14 | P0C0L4 | Complement C4-A | C4A |
| 15 | O75636 | Ficolin-3 | FCN3 |
| 16 | P02647 | Apolipoprotein A-I | APOA1 |
| 17 | P02766 | Transthyretin | TTR |
| 18 | P27169 | Serum paraoxonase/arylesterase 1 | PON1 |
| 19 | Q15485 | Ficolin-2 | FCN2 |
| 20 | Q03591 | Complement factor H-related protein 1 | CFHR1 |
| 21 | P02748 | Complement component C9 | C9 |
| 22 | P22352 | Glutathione peroxidase 3 | GPX3 |
| 23 | P01860 | Ig gamma-3 chain C region | IGHG3 |
| 24 | Q9BXR6 | Complement factor H-related protein 5 | CFHR5 |
| 25 | Q6UX53 | Methyltransferase-like protein 7B | METTL7B |
| 26 | P02774 | Vitamin D-binding protein | GC |
| 27 | P06681 | Complement C2 | C2 |
| 28 | P19823 | Inter-alpha-trypsin inhibitor heavy chain H2 | ITIH2 |
| 29 | P00734 | Prothrombin | F2 |
| 30 | P02649 | Apolipoprotein E | APOE |
| 31 | P02741 | C-reactive protein | CRP |
| 32 | P10909 | Clusterin | CLU |
| 33 | P01871 | Ig mu chain C region | IGHM |
| 34 | P01834 | Ig kappa chain C region | IGKC |
| 35 | O00187 | Mannan-binding lectin serine protease 2 | MASP2 |

### Validation of candidate biomarkers: ELISA analysis

The differential serum proteins identified by 2D-DIGE proteomics were validated by ELISA assays of the top 17 out of the 35 ranked proteins shown on Table 4. The ELISA results are shown in Table 5.

**Table 5**

| **Concentrations of 17 differentially expressed (in miligrams) protein with respect to serum volume (in dL) obtained by ELISA analysis. *p*-values were obtained by the Kruskal-Wallis test** | | | | | | | | | |
|---|---|---|---|---|---|---|---|---|---|
| **Gene name** | **POAG group mg/dL** | **PEXG group mg/dL** | **Control group mg/dL** | **FOLD CHANGE** | | | **Kruskal-Wallis Test (Non-parametric ANOVA)** | | |
| | | | | **POAG vs Control** | **PEXG vs Control** | **POAG vs PEXG** | **POAG vs Control** | **PEXG vs Control** | **POAG vs PEXG** |
| APOA4 | 9.95 ± 3.72 | 6.10 ± 1.65 | 3.81 ± 1.17 | 2,6 | 1,6 | 1,6 | *p* < 0.001 | *p* < 0.001 | *p* < 0.001 |
| C3 | 437.25 ± 97.62 | 333.15 ± 49.77 | 285.54 ± 45.38 | 1,5 | 1,2 | 1,3 | *p* < 0.001 | *p* < 0.05 | *p* < 0.001 |
| TF | 477.95 ± 162.65 | 303.78 ± 61.54 | 258.77 ± 55.34 | 1,8 | 1,2 | 1,6 | *p* < 0.001 | *p* > 0.05 | *p* < 0.001 |
| VTN | 117.39 ± 43.48 | 73.92 ± 25.42 | 51.86 ± 14.90 | 2,3 | 1,4 | 1,6 | *p* < 0.001 | *p* < 0.01 | *p* < 0.01 |
| TTR | 169.80 ± 61.84 | 111.12 ± 40.03 | 84.08 ± 25.70 | 2,0 | 1,3 | 1,5 | *p* < 0.001 | *p* < 0.05 | *p* < 0.001 |
| SERPINA1 | 296.12 ± 63.42 | 254.45 ± 57.39 | 198.33 ± 55.71 | 1,5 | 1,3 | 1,2 | *p* < 0.001 | *p* < 0.01 | *p* > 0.05 |
| FBLN1 | 29.33 ± 9.50 | 21.94 ± 8.09 | 14.71 ± 5.26 | 2,0 | 1,5 | 1,3 | *p* < 0.001 | *p* < 0.01 | *p* < 0.05 |
| APOA1 | 259.33 ± 59.32 | 212.32 ± 39.07 | 182.26 ± 34.42 | 1,4 | 1,2 | 1,2 | *p* < 0.001 | *p* < 0.05 | *p* < 0.05 |
| FCN3 | 117.14 ± 26.51 | 92.54 ± 22.56 | 85.31 ± 19.56 | 1,4 | 1,1 | 1,3 | *p* < 0.001 | *p* > 0.05 | *p* < 0.01 |
| CFH | 119.90 ±26.84 | 107.81 ± 25.73 | 91.87 ± 30.46 | 1,3 | 1,2 | 1,1 | *p* < 0.001 | *p* < 0.05 | *p* > 0.05 |
| ITIH4 | 614.22 ± 312.00 | 389.87 ± 196.29 | 352.53 ± 221.15 | 1,7 | 1,1 | 1,6 | *p* < 0.01 | *p* > 0.05 | *p* < 0.05 |
| APOL1 | 9.01 ± 3.07 | 8.21 ± 2.76 | 6.05 ± 2.16 | 1,5 | 1,4 | 1,1 | *p* < 0.001 | *p* < 0.05 | *p* > 0.05 |
| ALB | 3339.11 ± 1021.24 | 3885.80 ± 913.34 | 3609.80 ± 908.36 | 0,9 | 1,1 | 0,9 | *p* > 0.05 | *p* > 0.05 | *p* > 0.05 |
| SERPINC1 | 78.54 ± 10.40 | 79.45 ± 12.99 | 82.01 ± 12.72 | 1,0 | 1,0 | 1,0 | *p* > 0.05 | *p* > 0.05 | *p* > 0.05 |
| IGHG2 | 770.19 ± 253.78 | 809.27 ± 290.21 | 1136.81 ± 354.62 | 0,7 | 0,7 | 1,0 | *p* < 0.001 | *p* < 0.01 | *p* > 0.05 |
| C4A | 0.16 ± 0.05 | 0.15 ± 0.04 | 0.18 ± 0.03 | 0,9 | 0,8 | 1,0 | *p* > 0.05 | *p* > 0.05 | *p* > 0.05 |
| APCS | 17.08 ± 12.13 | 19.20 ± 14.53 | 19.43 ± 12.96 | 0,9 | 1,0 | 0,9 | *p* > 0.05 | *p* > 0.05 | *p* > 0.05 |

The concentration (mg protein/dL serum) (mean value ± SD) found for each of the 17 tested proteins in POAG, PEXG, and control samples is presented, as well as their fold-change and Kruskal-Wallis Test (non-parametric ANOVA) values between groups (i.e., POAG/control, PEXG/control and POAG/PEXG). Four proteins: ALB, SERPINC1, C4A and APCS, displayed no significant differences among the three sample groups (i.e., POAG, PEXG and control). The rest of the proteins investigated were found in higher concentrations in glaucoma (POAG and/or PEXG) cases compared to control, except in the case of IGHG2 protein, which exhibited a lower concentration in the two glaucoma groups compared to controls.

Six proteins: APOA4, C3, TTR, VTN, FBLN1 and APOA1, were found in higher concentrations (1.2- to 2.6-fold) in POAG and PEXG relative to control cases (i.e., POAG *vs* control, *p*<0.001, PEXG *vs* control: APOA4, *p*<0.001; VTN and FBLN1, *p*<0.01; C3, TTR and APOA1, *p*<0.05). These concentrations were also significantly elevated (1.2- to 1.6-fold) among POAG when compared to PEXG cases (i.e., APOA4, C3, and TTR, *p*<0.001; VTN, *p*<0.01; FBLN1 and APOA1 p<0.05).

The proteins SERPINA1, CFH, and APOL1, were found in uppermost concentrations (1.2- to 1.5-fold) in both POAG and PEXG cases when compared to controls (POAG *vs* control, *p*<0.001, PEXG *vs* control, SERPINA1, *p*<0.01; CFH and APOL1, p<0.05). None of these three latter proteins showed significant differences between POAG and PEXG groups (*p*>0.05).

The proteins TF, FCN3, and ITIH4, were found in higher concentrations (1.4- to 1.8 fold) among POAG relative to control cases (i.e., TF and FCN3, *p*<0.001; ITIH4, *p*<0.01). These concentrations were also significantly elevated (1.3- to 1.6-fold among POAG when compared to PEXG cases (i.e., TF, *p*<0.001; FCN3, *p*<0.01; ITIH4, *p*<0.05), but no differences were found between PEXG and control (*p*>0.05).

The protein IGHG2 was found in lower concentrations in POAG (0.7 fold) and PEXG (0.7 fold) cases when compared with controls (POAG, *p*<0.001; PEXG, *p*<0.01). No significant differences was found between POAG and PEXG groups (*p*>0.05).

### Machine learning models

Once the concentration of each protein had been obtained, ROC curve analysis was performed with each of the markers individually by means of Naive Bayes machine learning algorithm. Machine learning statistics were carried out using the Orange Canvas statistic package, version 2.6 (http://orange.biolab.si). Table 6 shows correct assignment (CA), sensitivity, specificity and area under the curve (AUC) data obtained. The discrimination power for each of the proteins was ranked based on their AUC values, which indicates the accuracy of the test.

Further ROC curve analysis by means of Naive Bayes machine learning algorithm was performed for some particular combinations of biomarkers. Table 7 shows the sensitivity, specificity and area under the curve (AUC) data obtained for each panel of biomarkers. Interestingly, panels 1, 4, 7, 8 show an excellent discrimination power between the PEXG group and the control group; panels 2, 7, 8 and 14 show an excellent discrimination power between the POAG group and the control group; panels 5, 6, 7 and 8 show an excellent discrimination power between the POAG group and the PEXG group; and panels 3, 7, 8 and 13 show an excellent discrimination power between the POAG group, the PEXG group and the control group.

**Table 6**

| **Results of individual biomarker power discrimination obtained with Naïve Bayes algorithm in the classification of POAG, PEXC and control serum samples.** | | | | | | | | | | | | | | | | |
|---|---|---|---|---|---|---|---|---|---|---|---|---|---|---|---|---|
| **Gene name** | **POAG vs PEXG vs Control** | | | | **POAG vs Control** | | | | **PEXG vs Control** | | | | **POAG vs PEXG** | | | |
| | CA^{a} | Sens.^{b} | Spec.^{c} | AUC^{d} | CA | Sens. | Spec. | AUC | CA | Sens. | Spec. | AUC | CA | Sens. | Spec. | AUC |
| APOA4 | 0.70 | 0.93 | 0.93 | 0.89 | 0.95 | 0.93 | 0.97 | 0.99 | 0.90 | 0.93 | 0.86 | 0.89 | 64,0 | 0.63 | 0.65 | 0.77 |
| C3 | 0.69 | 0.76 | 0.91 | 0.85 | 0.86 | 0.90 | 0.83 | 0.97 | 0.79 | 0.76 | 0.83 | 0.89 | 0.75 | 0.67 | 0.83 | 0.72 |
| TF | 0.72 | 0.90 | 0.86 | 0.85 | 0.88 | 0.93 | 0.83 | 0.97 | 0.84 | 0.90 | 0.79 | 0.84 | 0.71 | 0.57 | 0.86 | 0.72 |
| VTN | 0.65 | 0.90 | 0.85 | 0.80 | 0.91 | 0.93 | 0.90 | 0.96 | 0.76 | 0.79 | 0.72 | 0.86 | 0.61 | 0.47 | 0.76 | 0.68 |
| TTR | 0.65 | 0.83 | 0.85 | 0.81 | 0.85 | 0.83 | 0.87 | 0.95 | 0.79 | 0.83 | 0.76 | 0.79 | 0.69 | 0.63 | 0.76 | 0.70 |
| SERPINA1 | 0.61 | 0.93 | 0.80 | 0.77 | 0.90 | 0.93 | 0.87 | 0.92 | 0.81 | 0.86 | 0.76 | 0.89 | 0.59 | 0.50 | 0.69 | 0.55 |
| FBLN1 | 0.62 | 0.53 | 0.84 | 0.76 | 0.86 | 0.93 | 0.79 | 0.94 | 0.81 | 0.79 | 0.83 | 0.84 | 0.64 | 0.53 | 0.76 | 0.52 |
| APOA1 | 0.59 | 0.76 | 0.78 | 0.75 | 0.78 | 0.79 | 0.77 | 0.88 | 0.74 | 0.76 | 0.72 | 0.85 | 0.59 | 0.40 | 0.79 | 0.53 |
| FCN3 | 0.64 | 0.65 | 0.85 | 0.74 | 0.80 | 0.86 | 0.73 | 0.86 | 0.74 | 0.65 | 0.83 | 0.73 | 0.66 | 0.57 | 0.76 | 0.62 |
| CFH | 0.61 | 0.79 | 0.86 | 0.72 | 0.80 | 0.79 | 0.80 | 0.86 | 0.83 | 0.79 | 0.86 | 0.81 | 0.54 | 0.47 | 0.62 | 0.51 |
| ITIH4 | 0.47 | 0.79 | 0.58 | 0.63 | 0.71 | 0.79 | 0.63 | 0.77 | 0.59 | 0.76 | 0.41 | 0.56 | 0.63 | 0.47 | 0.79 | 0.55 |
| APOL1 | 0.45 | 0.65 | 0.76 | 0.61 | 0.68 | 0.69 | 0.67 | 0.80 | 0.71 | 0.65 | 0.76 | 0.77 | 0.47 | 0.53 | 0.41 | 0.27 |
| ALB | 0.44 | 0.28 | 0.86 | 0.55 | 0.37 | 0.28 | 0.52 | 0.35 | 0.72 | 0.65 | 0.79 | 0.70 | 0.64 | 0.57 | 0.72 | 0.64 |
| SERPINC1 | 0.35 | 0.45 | 0.78 | 0.49 | 0.64 | 0.45 | 0.83 | 0.52 | 0.67 | 0.55 | 0.79 | 0.63 | 0.37 | 0.40 | 0.34 | 0.32 |
| IGHG2 | 0.34 | 0.38 | 0.63 | 0.45 | 0.54 | 0.59 | 0.50 | 0.58 | 0.43 | 0.38 | 0.48 | 0.38 | 0.52 | 0.37 | 0.69 | 0.37 |
| C4A | 0.26 | 0.45 | 0.63 | 0.42 | 0.46 | 0.45 | 0.47 | 0.43 | 0.60 | 0.62 | 0.59 | 0.56 | 0.36 | 0.47 | 0.24 | 0.25 |
| APCS | 0.24 | 0.52 | 0.63 | 0.33 | 0.36 | 0.48 | 0.23 | 0.21 | 0.52 | 0.62 | 0.41 | 0.49 | 0.34 | 0.43 | 0.24 | 0.32 |

| | | | | | | | | | | | | | | | | |
|---|---|---|---|---|---|---|---|---|---|---|---|---|---|---|---|---|
| ^{a}CA, correct assignment. ^{b}Sens., sensitivity. ^{c}Spec., specificity. ^{d}AUC, area under the curve. | | | | | | | | | | | | | | | | |

**Table 7**

| **Results of panels of biomarkers power discrimination obtained with Naive Bayes algorithm in the classification of POAG, PEXG and control serum samples.** | | | | | | |
|---|---|---|---|---|---|---|
| Panel | Panel # | # markers | Groups assigned | Sens. (%) | Spec. (%) | AUC (%) |
| APOA4, IGHG2, APOL1, and C4A | 1 | 4 | PEXG vs Control | 77.78 | 90.00 | 91.60 |
| APOA4, C3, C4A, APCS, and SERPINA1 | 2 | 5 | POAG vs Control | 96.67 | 96.67 | 99.75 |
| APOA4, C3, TF, APOL1, IGHG2, and C4A | 3 | 6 | POAG vs PEXG vs Control | 92 | 83 | 96 |
| APOA4, C4A, APOL1, IGHG2, and C3 | 4 | 5 | PEXG vs Control | 76.67 | 87.78 | 92.47 |
| APOA4, C3, TF, C4A | 5 | 4 | POAG vs PEXG | 75.56 | 80.00 | 83.33 |
| APOA4, C3, and TF | 6 | 3 | POAG vs PEXG | 72.22 | 85.56 | 84.20 |
| APOA4, C3, TF, VTN, TTR, SERPINA1, FBLN1, APOA1, FCN3, CFH, ITIH4, APOL1, ALB, SERPINC1, IGHG2, C4A, and APCS | 7 | 17 | POAG vs PEXG vs Control | 87.78 | 95.00 | 92.16 |
| | | 17 | POAG vs Control | 97.78 | 92.22 | 99.26 |
| | | 17 | PEXG vs Control | 81.11 | 94.44 | 96.42 |
| | | 17 | POAG vs PEXG | 65.56 | 73.33 | 78.89 |
| C3, TF, VTN, TTR, SERPINA1, FBLN1, APOA1, FCN3, CFH, ITIH4, APOL1, ALB, SERPINC1, IGHG2, and APCS | 8 | 15 | POAG vs PEXG vs Control | 88.89 | 95.00 | 91.13 |
| | | 15 | POAG vs Control | 96.67 | 88.89 | 98.77 |
| | | 15 | PEXG vs Control | 81.11 | 94.44 | 95.68 |
| | | 15 | POAG vs PEXG | 62.22 | 74.44 | 75.37 |
| ALB, SERPINC1, IGHG2, C4A, and APCS | 9 | 5 | POAG vs PEXG vs Control | 62.22 | 72.22 | 62.51 |
| ALB, SERPINC1, IGHG2, C4A, APCS, ITIH4, and APOL1 | 10 | 7 | POAG vs PEXG vs Control | 74.44 | 83.89 | 77.49 |
| IGHG2, C4A, APCS, and ITIH4 | 11 | 4 | PEXG vs Control | 47.78 | 70.00 | 60.86 |
| SERPINC1, IGHG2, C4A, APCS, and ITIH4 | 12 | 5 | PEXG vs Control | 55.56 | 71.11 | 71.60 |
| C3, SERPINC1, TF, APOL1 | 13 | 4 | POAG vs PEXG vs Control | 88.89 | 95.00 | 91.13 |
| C3, SERPINC1, APCS, SERPINA1 | 14 | 4 | POAG vs Control | 96.67 | 88.89 | 98.77 |

Based on the concentration found for each of the 17 proteins in serum samples from POAG, PEXG and control patients, a stepwise discriminant analysis with a significance cutoff of 0.05 was used to determine which variables (proteins) were the most important to differentiate between the groups. As a result of this feature-subset subtraction, six protein biomarkers appear to be significant in the discrimination of the studied groups. This panel included C3, APOA4, C4A, TF, IGHG2 and APOL1 proteins, which were accordingly selected to generate multivariate predictive models. To this end, inventors performed training using random sampling with 75% of the data and distinct machine learning algorithms, such as Naive Bayes, k-Nearest Neighbor, Random Forest, Classification Tree, or an ensemble of Support Vector Machine and Naive Bayes algorithms (AdaBoost.M1). Subsequently, the classification method was validated using the remaining 25% of data.

As a result of this analysis, it was found that the best classification efficiency was obtained when using the full panel of six markers and principally training the data with Naive Bayes and the Adaboost.M1 (ensemble of two algorithms: Support Vector Machine and Naive Bayes) with correct assignment values of 79.87% and 81.17% respectively, and comparing all three study groups (Table 8).

**Table 8**

| **Predictive yield between the PEGX, POAG and control groups obtained in the validation study of a 6 marker panel (C3, APOA4, C4A, TF, IGHG2 and APOL1) implementing different machine learning algorithms Evaluation Results** | | | | | |
|---|---|---|---|---|---|
| | Method | CA^{a} | Sens.^{b} | Spec.^{c} | AUC^{d} |
| 1 | Naive Bayes | 0.7987 | 0.8214 | 0.9286 | 0.9583 |
| 2 | kNN | 0.6948 | 0.8214 | 0.8673 | 0.9228 |
| 3 | Classification Tree | 0.7078 | 0.8036 | 0.8571 | 0.8154 |
| 4 | Random Forest | 0.7078 | 0.8571 | 0.8367 | 0.9042 |
| 5 | AdaBoost.M1 | 0.8117 | 0.8214 | 0.9490 | 0.9210 |

| | | | | | |
|---|---|---|---|---|---|
| ^{a}CA, correct assignment. ^{b}Sens., sensitivity. ^{c}Spec., specificity. ^{d}AUC, area under the curve. | | | | | |

The confusion matrix that resulted from the validation of the classifier, generated by means of Random Naive Bayes, is showed in Table 8. In general terms, the diagnostic efficiency reached 81% (correct assignment, CA), and a specificity (Spec.) of 83% and sensitivity (Sens.) of 92% were obtained (see Table 9).

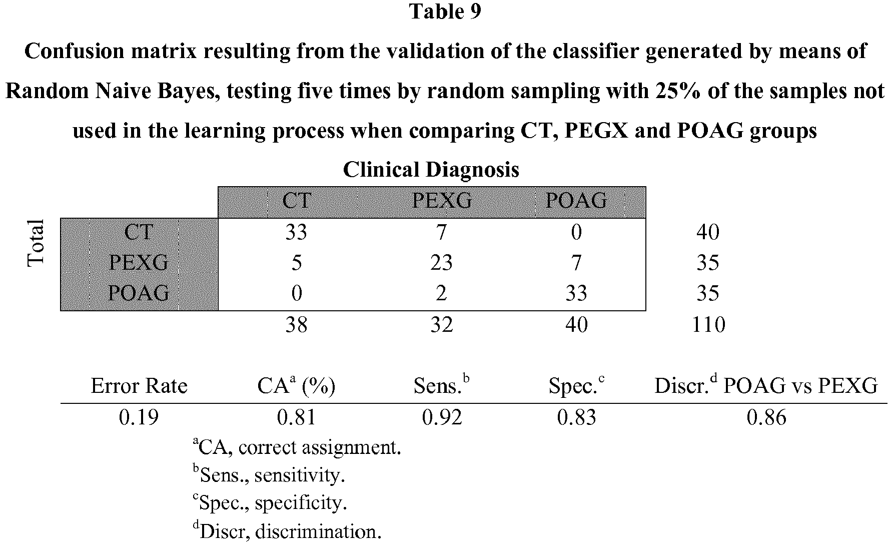

In this fashion, inventors managed to correctly classify 83% of the control samples and 92% of the cases with POAG or PEXG pathology. It is noteworthy that 100% efficacy was reached when discriminating the POAG group from the control group, since no control sample was classified as POAG or *viceversa.* The six-marker panel also exhibited correct discrimination of POAG and PEGX in 86% of the pathological samples, while the least-precise prediction was the diagnosis of the PEGX group, with an overall correct assignment of 66%.

## Claims

1. An *in vitro* method for the diagnosis of a glaucoma selected from primary open angle glaucoma and pseudoexfoliation glaucoma in a subject comprising
(i) determining in a sample from said subject the expression level of apolipoprotein A-IV and
(ii) comparing the expression level of apolipoprotein A-IV with a reference value
and, wherein,
a) if the subject suffers from pseudoexfoliation syndrome, an increase in the expression level of apolipoprotein A-IV compared to a reference value is indicative of pseudoexfoliation glaucoma, or
b) if the subject does not suffer from pseudoexfoliation syndrome, an increase in the expression level of apolipoprotein A-IV compared to a reference value is indicative of primary open angle glaucoma.

2. The method according to claim 1, further comprising determining in a sample from said subject the expression level of at least one additional biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, apolipoprotein L1, serum albumin and combinations thereof wherein
a) if the subject suffers from pseudoexfoliation syndrome,
- an increase in the expression level of at least one biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, apolipoprotein L1, and serum albumin compared to a reference value
is indicative of pseudoexfoliation glaucoma, or
b) if the subject does not suffer from pseudoexfoliation syndrome,
- an increase in the expression level of at least one biomarker selected from the group consisting of complement C3, serotransferrin, vitronectin, transthyretin, alpha-1 antitrypsin, fibulin-1, apolipoprotein A-I, ficolin-3, complement factor H, inter-alpha-trypsin inhibitor heavy chain H4, and apolipoprotein L1 compared to a reference value, and/or
- a decrease in the expression level of serum albumin compared to a reference value,
is indicative of primary open angle glaucoma.

3. The method according to any one of claims 1 or 2, further comprising determining in a sample from said subject the expression level of at least one additional biomarker selected from the group consisting of Ig gamma-2 chain C region, antithrombin-III, complement C4A, serum amyloid P-component and combinations thereof.

4. The method according to any one of claims 1 or 2, comprising determining the expression level of biomarkers apolipoprotein A-IV, complement C3 and serotransferrin.

5. The method according to any one of claims 1 to 3, comprising determining the expression level of biomarkers apolipoprotein A-IV, complement C4A, Ig gamma-2 chain C region and apolipoprotein L1.

6. The method according to any one of claims 1 to 3, comprising determining the expression level of biomarkers apolipoprotein A-IV, complement C4A, complement C3, serum amyloid P-component and alpha-1 antitrypsin.

7. The method according to any one of claims 1 to 3, comprising determining the expression level of biomarkers apolipoprotein A-IV, complement C4A, complement C3, serotransferrin, apolipoprotein L1 and Ig gamma-2 chain C region.

8. The method according to any one of claims 1 to 3, comprising determining the expression level of all of the biomarkers from Table 1 and the additional biomarkers Ig gamma-2 chain C region, antithrombin-III, complement C4A and serum amyloid P-component wherein Table 1 is
| **Protein name** | **Gene name** |
|---|---|
| Apolipoprotein A-IV | APOA4 |
| Complement C3 | C3 |
| Serotransferrin | TF |
| Vitronectin | VTN |
| Transthyretin | TTR |
| Alpha-1 antitrypsin | SERPINA1 |
| Fibulin-1 | FBLN1 |
| Apolipoprotein A-I | APOA1 |
| Ficolin-3 | FCN3 |
| Complement factor H | CFH |
| Inter-alpha-trypsin inhibitor heavy chain H4 | ITIH4 |
| Apolipoprotein L1 | APOL1 |
| Serum albumin | ALB |

9. The method according to any one of claims 1 to 8, wherein the sample is a bodily fluid sample.

10. The method according to claim 9, wherein the bodily fluid sample is blood, plasma or blood serum.

11. A kit comprising a reagent for determining the expression level of apolipoprotein A-IV and an additional biomarker selected from the group consisting of complement C3, serotransferrin, vitronectin, fibulin-1, ficolin-3, complement factor H, inter-alpha-trypsin inhibitor heavy chain H4, apolipoprotein L1, serum albumin and combinations thereof wherein the reagent for determining the expression level is selected from the group consisting of:
(a) an antibody, an aptamer, or a fragment thereof that specifically binds to the biomarker protein; and
(b) a reagent suitable for determining the expression level of the biomarker by means of a multiple reaction monitoring (MRM) assay, said reagent being a isotope-labeled peptide obtainable by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin and endoproteinase GluC.

12. The kit according to claim 11, further comprising a reagent for determining the expression level of an additional biomarker, wherein said additional biomarker is selected from the group consisting of Ig gamma-2 chain C region, antithrombin-III, complement C4A, serum amyloid P-component and combinations thereof and wherein the reagent for determining the expression level is selected from the group consisting of:
(a) an antibody, an aptamer, or a fragment thereof that specifically binds to the biomarker protein; and
(b) a reagent suitable for determining the expression level of the biomarker by means of a multiple reaction monitoring (MRM) assay, said reagent being a isotope-labeled peptide obtainable by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin and endoproteinase GluC.

13. The kit according to any one of claims 11 or 12, wherein said reagents are antibodies, or fragments thereof having the ability to recognize the corresponding antigen, that specifically bind to said biomarkers.

14. Use of a kit comprising a reagent for determining the expression level of at least apolipoprotein A-IV and optionally at least one biomarker selected from the biomarkers listed in Table 1 for diagnosis of a glaucoma selected from primary open angle glaucoma and pseudoexfoliation glaucoma, wherein the reagent for determining the expression level is selected from the group consisting of:
(a) an antibody, an aptamer, or a fragment thereof that specifically binds to the biomarker protein; and
(b) a reagent suitable for determining the expression level of the biomarker by means of a multiple reaction monitoring (MRM) assay, said reagent being a isotope-labeled peptide obtainable by enzymatic digestion of said biomarker protein with a digestive enzyme selected from the group consisting of trypsin, papain, endoproteinase LysC, endoproteinase ArgC, staph aureus V8, chymotrypsin, Asp-N, Asn-C, pepsin and endoproteinase GluC.

15. Use of a kit according to claim 14, wherein said reagent is an antibody, or a fragment thereof having the ability to recognize the corresponding antigen, that specifically binds to said biomarker.

## Patentansprüche

1. *In vitro* Verfahren zur Diagnose eines Glaukoms, ausgewählt aus primärem Offenwinkelglaukom und Pseudoexfoliationsglaukom, bei einem Subjekt, umfassend
(i) Bestimmen des Expressionsniveaus von Apolipoprotein A-IV in einer Probe von dem Subjekt, und
(ii) Vergleichen des Expressionsniveaus von Apolipoprotein A-IV mit einem Referenzwert
und, wobei,
a) wenn das Subjekt an Pseudoexfoliationssyndrom leidet, eine Zunahme des Expressionsniveaus von Apolipoprotein A-IV im Vergleich zu einem Referenzwert indikativ für Pseudoexfoliationsglaukom ist oder
b) wenn das Subjekt nicht an Pseudoexfoliationssyndrom leidet, eine Zunahme des Expressionsniveaus von Apolipoprotein A-IV im Vergleich zu einem Referenzwert indikativ für primäres Offenwinkelglaukom ist.

2. Verfahren nach Anspruch 1, ferner umfassend ein Bestimmen des Expressionsniveaus von mindestens einem zusätzlichen Biomarker ausgewählt aus der Gruppe bestehend aus Komplement C3, Serotransferrin, Vitronectin, Transthyretin, alpha-1-Antitrypsin, Fibulin-1, Apolipoprotein A-I, Ficolin-3, Komplementfaktor H, schwere Kette eines Inter-Alpha-Trypsin-Inhibitors H4, Apolipoprotein L1, Serumalbumin und Kombinationen davon, in einer Probe von dem Subjekt, wobei
a) wenn das Subjekt an Pseudoexfoliationssyndrom leidet,
- eine Zunahme des Expressionsniveaus von mindestens einem Biomarker ausgewählt aus der Gruppe bestehend aus Komplement C3, Serotransferrin, Vitronectin, Transthyretin, alpha-1-Antitrypsin, Fibulin-1, Apolipoprotein A-I, Ficolin-3, Komplementfaktor H, schwere Kette eines Inter-Alpha-Trypsin-Inhibitors H4, Apolipoprotein L1 und Serumalbumin im Vergleich zu einem Referenzwert
indikativ für Pseudoexfoliationsglaukom ist, oder
b) wenn das Subjekt nicht an Pseudoexfoliationssyndrom leidet,
- eine Zunahme des Expressionsniveaus von mindestens einem Biomarker ausgewählt aus der Gruppe bestehend aus Komplement C3, Serotransferrin, Vitronectin, Transthyretin, alpha-1-Antitrypsin, Fibulin-1, Apolipoprotein A-I, Ficolin-3, Komplementfaktor H, schwere Kette eines Inter-Alpha-Trypsin-Inhibitors H4 und Apolipoprotein L1 im Vergleich zu einem Referenzwert und/oder
- eine Abnahme des Expressionsniveaus von Serumalbumin im Vergleich zu einem Referenzwert
indikativ für primäres Offenwinkelglaukom ist.

3. Verfahren nach einem der Ansprüche 1 oder 2, ferner umfassend ein Bestimmen des Expressionsniveaus von mindestens einem zusätzlichen Biomarker, ausgewählt aus der Gruppe bestehend aus C-Region der Ig-Gamma-2 Kette, Antithrombin-III, Komplement C4A, Serumamyloid-P-Komponente und Kombinationen davon in einer Probe von dem Subjekt.

4. Verfahren nach einem der Ansprüche 1 oder 2, umfassend ein Bestimmen des Expressionsniveaus der Biomarker Apolipoprotein A-IV, Komplement C3 und Serotransferrin.

5. Verfahren nach einem der Ansprüche 1 bis 3, umfassend ein Bestimmen des Expressionsniveaus der Biomarker Apolipoprotein A-IV, Komplement C4A, C-Region der Ig-Gamma-2 Kette und Apolipoprotein L1.

6. Verfahren nach einem der Ansprüche 1 bis 3, umfassend ein Bestimmen des Expressionsniveaus der Biomarker Apolipoprotein A-IV, Komplement C4A, Komplement C3, Serumamyloid-P-Komponente und alpha-1-Antitrypsin.

7. Verfahren nach einem der Ansprüche 1 bis 3, umfassend ein Bestimmen des Expressionsniveaus der Biomarker Apolipoprotein A-IV, Komplement C4A, Komplement C3, Serotransferrin, Apolipoprotein L1 und C-Region der Ig-Gamma-2 Kette.

8. Verfahren nach einem der Ansprüche 1 bis 3, umfassend ein Bestimmen des Expressionsniveaus aller Biomarker aus Tabelle 1 und der zusätzlichen Biomarker C-Region der Ig-Gamma-2 Kette, Antithrombin-III, Komplement C4A und Serumamyloid-P-Komponente, wobei Tabelle 1
| **Proteinname** | **Genname** |
|---|---|
| Apolipoprotein A-IV | APOA4 |
| Komplement C3 | C3 |
| Serotransferrin | TF |
| Vitronectin | VTN |
| Transthyretin | TTR |
| alpha-1-Antitrypsin | SERPINA1 |
| Fibulin-1 | FBLN1 |
| Apolipoprotein A-I | APOA1 |
| Ficolin-3 | FCN3 |
| Komplementfaktor H | CFH |
| schwere Kette eines Inter-Alpha-Trypsin-Inhibitors H4 | ITIH4 |
| Apolipoprotein L1 | APOL1 |
| Serumalbumin | ALB |
ist.

9. Verfahren nach einem der Ansprüche 1 bis 8, wobei die Probe eine Körperflüssigkeitsprobe ist.

10. Verfahren nach Anspruch 9, wobei die Körperflüssigkeitsprobe Blut, Plasma oder Blutserum ist.

11. Kit, umfassend ein Reagenz zum Bestimmen des Expressionsniveaus von Apolipoprotein A-IV und einem zusätzlichen Biomarker ausgewählt aus der Gruppe bestehend aus Komplement C3, Serotransferrin, Vitronectin, Fibulin-1, Ficolin-3, Komplementfaktor H, schwere Kette eines Inter-Alpha-Trypsin-Inhibitors H4, Apolipoprotein L1, Serumalbumin und Kombinationen davon, wobei das Reagenz zum Bestimmen des Expressionsniveaus ausgewählt ist aus der Gruppe bestehend aus:
(a) einem Antikörper, einem Aptamer oder einem Fragment davon, der/das spezifisch an das Biomarkerprotein bindet; und
(b) einem Reagenz, das geeignet ist das Expressionsniveau des Biomarkers mittels eines Multiple Reaction Monitoring (MRM) Assays zu bestimmen, wobei das Reagenz ein Isotop-markiertes Peptid ist, das durch enzymatische Verdauung des Biomarkerproteins mit einem Verdauungsprotein, ausgewählt aus der Gruppe bestehend aus Trypsin, Papain, Endoproteinase LysC, Endoproteinase ArgC, Staph Aureus V8, Chymotrypsin, Asp-N, Asn-C, Pepsin und Endoproteinase GluC, erhältlich ist.

12. Kit nach Anspruch 11, weiterhin umfassend ein Reagenz zum Bestimmen des Expressionsniveaus eines zusätzlichen Biomarkers, wobei der zusätzliche Biomarker ausgewählt ist aus der Gruppe bestehend aus C-Region der Ig-Gamma-2 Kette, Antithrombin-III, Komplement C4A, Serumamyloid-P-Komponente und Kombinationen davon, und wobei das Reagenz zum Bestimmen des Expressionsniveaus ausgewählt ist aus der Gruppe bestehend aus:
(a) einem Antikörper, einem Aptamer oder einem Fragment davon, der/das spezifisch an das Biomarkerprotein bindet; und
(b) einem Reagenz, das geeignet ist das Expressionsniveau des Biomarkers mittels eines Multiple Reaction Monitoring (MRM) Assays zu bestimmen, wobei das Reagenz ein Isotop-markiertes Peptid ist, das durch enzymatische Verdauung des Biomarkerproteins mit einem Verdauungsprotein, ausgewählt aus der Gruppe bestehend aus Trypsin, Papain, Endoproteinase LysC, Endoproteinase ArgC, Staph Aureus V8, Chymotrypsin, Asp-N, Asn-C, Pepsin und Endoproteinase GluC, erhältlich ist.

13. Kit nach einem der Ansprüche 11 oder 12, wobei die Reagenzien Antikörper oder Fragmente davon sind, die die Fähigkeit aufweisen das korrespondierende Antigen zu erkennen, das spezifisch an die Biomarker bindet.

14. Verwendung eines Kits, umfassend ein Reagens zum Bestimmen des Expressionsniveaus von mindestens Apolipoprotein A-IV und gegebenenfalls mindestens einem Biomarker ausgewählt aus den Biomarken, die in Tabelle 1 aufgelistet sind, zur Diagnose eines Glaukoms, ausgewählt aus primärem Offenwinkelglaukom und Pseudoexfoliationsglaukom, wobei das Reagenz zum Bestimmen des Expressionsniveaus ausgewählt ist aus der Gruppe bestehend aus:
(a) einem Antikörper, einem Aptamer oder einem Fragment davon, der/das spezifisch an das Biomarkerprotein bindet; und
(b) einem Reagenz, das geeignet ist das Expressionsniveau des Biomarkers mittels eines Multiple Reaction Monitoring (MRM) Assays zu bestimmen, wobei das Reagenz ein Isotop-markiertes Peptid ist, das durch enzymatische Verdauung des Biomarkerproteins mit einem Verdauungsprotein ausgewählt aus der Gruppe bestehend aus Trypsin, Papain, Endoproteinase LysC, Endoproteinase ArgC, Staph Aureus V8, Chymotrypsin, Asp-N, Asn-C, Pepsin und Endoproteinase GluC erhältlich ist.

15. Verwendung eines Kits nach Anspruch 14, wobei das Reagenz ein Antikörper oder ein Fragment davon ist, der/das die Fähigkeit aufweist das korrespondierende Antigen zu erkennen, das spezifisch an den Biomarker bindet.

## Revendications

1. Un procédé *in vitro* pour le diagnostic d'un glaucome figurant parmi le glaucome primaire à angle ouvert et le glaucome à pseudo-exfoliation chez un sujet, comprenant
(i) le fait de déterminer dans un échantillon provenant dudit sujet le niveau d'expression de l'apolipoprotéine A-IV et
(ii) le fait de comparer le niveau d'expression de l'apolipoprotéine A-IV avec une valeur de référence
et dans lequel,
a) si le sujet présente un syndrome de pseudo-exfoliation, une augmentation du niveau d'expression de l'apolipoprotéine A-IV par rapport à une valeur de référence est indicatrice d'un glaucome à pseudo-exfoliation, ou
b) si le sujet ne souffre pas d'un syndrome de pseudo-exfoliation, une augmentation du niveau d'expression de l'apolipoprotéine A-IV par rapport à une valeur de référence est indicatrice d'un glaucome primaire à angle ouvert.

2. Le procédé selon la revendication 1, comprenant en outre le fait de déterminer dans un échantillon provenant dudit sujet le niveau d'expression d'au moins un biomarqueur supplémentaire choisi dans le groupe constitué par le complément C3, la sérotransferrine, la vitronectine, la transthyrétine, l'alpha-1 antitrypsine, la fibuline-1, l'apolipoprotéine AI, la ficoline-3, le facteur H du complément, la chaîne lourde H4 de l'inhibiteur de l'inter-alpha-trypsine, l'apolipoprotéine L1, l'albumine sérique et les combinaisons de ceux-ci, dans lequel
a) si le sujet souffre du syndrome de pseudo-exfoliation,
- une augmentation du niveau d'expression d'au moins un biomarqueur choisi dans le groupe constitué du complément C3, de la sérotransferrine, de la vitronectine, de la transthyrétine, de l'alpha-1 antitrypsine, de la fibuline-1, de l'apolipoprotéine A-I, de la ficoline-3, du facteur H du complément, de la chaîne lourde H4 de l'inhibiteur de l'inter-alpha-trypsine, de l'apolipoprotéine L1 et de l'albumine sérique, en comparaison avec une valeur de référence
est indicatrice d'un glaucome à pseudo-exfoliation, ou
b) si le sujet ne souffre pas du syndrome de pseudo-exfoliation,
- une augmentation du niveau d'expression d'au moins un biomarqueur choisi dans le groupe constitué du complément C3, de la sérotransferrine, de la vitronéctine, de la transthyrétine, de l'alpha-1 antitrypsine, de la fibuline-1, de l'apolipoprotéine A-I, de la ficoline-3, du facteur H du complément, de la chaîne lourde H4 de l'inhibiteur de l'inter-alpha-trypsine et de l'apolipoprotéine L1 en comparaison avec une valeur de référence, et/ou
- une diminution du niveau d'expression de l'albumine sérique en comparaison avec une valeur de référence,
est indicatrice d'un glaucome primaire à angle ouvert.

3. Le procédé selon l'une quelconque des revendications 1 ou 2, comprenant en outre le fait de déterminer dans un échantillon provenant dudit sujet le niveau d'expression d'au moins un biomarqueur supplémentaire choisi dans le groupe constitué par la région C de la chaîne gamma-2 Ig, l'antithrombine-III, le complément C4A, le composant amyloïde P sérique et les combinaisons de ceux-ci.

4. Le procédé selon l'une quelconque des revendications 1 ou 2, comprenant le fait de déterminer le niveau d'expression des biomarqueurs apolipoprotéine A-IV, complément C3 et sérotransferrine.

5. Le procédé selon l'une quelconque des revendications 1 à 3, comprenant le fait de déterminer le niveau d'expression des biomarqueurs apolipoprotéine A-IV, complément C4A, région C de la chaîne gamma-2 Ig et apolipoprotéine L1.

6. Le procédé selon l'une quelconque des revendications 1 à 3, comprenant le fait de déterminer le niveau d'expression des biomarqueurs apolipoprotéine A-IV, complément C4A, complément C3, composant amyloïde P sérique et alpha-1 antitrypsine.

7. Le procédé selon l'une quelconque des revendications 1 à 3, comprenant le fait de déterminer le niveau d'expression des biomarqueurs apolipoprotéine A-IV, complément C4A, complément C3, sérotransferrine, apolipoprotéine L1 et région C de la chaîne gamma-2 Ig.

8. Le procédé selon l'une quelconque des revendications 1 à 3, comprenant le fait de déterminer le niveau d'expression de tous les biomarqueurs du tableau 1 et des biomarqueurs supplémentaires région C de la chaîne gamma-2 Ig, antithrombine-III, complément C4A et composant amyloïde P sérique, le Tableau 1 étant
| **Nom de la protéine** | **Nom du gène** |
|---|---|
| Apolipoprotéine A-IV | APOA4 |
| Complément C3 | C3 |
| Serotransferrine | TF |
| Vitronectine | VTN |
| Transthyrétine | TTR |
| Alpha-1 antitrypsine | SERPINA1 |
| Fibuline-1 | FBLN1 |
| Apolipoprotéine A-1 | APOA1 |
| Ficoline-3 | FCN3 |
| Facteur H du complément | CFH |
| chaîne lourde H4 de l'inhibiteur de l'inter-alpha-trypsine | ITIH4 |
| Apolipoprotéine L1 | APOL1 |
| Albumine sérique | ALB |

9. Le procédé selon l'une quelconque des revendications 1 à 8, dans lequel l'échantillon est un échantillon de fluide corporel.

10. Le procédé selon la revendication 9, dans lequel l'échantillon de fluide corporel est du sang, du plasma ou du sérum sanguin.

11. Un kit comprenant un réactif pour déterminer le niveau d'expression de l'apolipoprotéine A-IV et un biomarqueur supplémentaire choisi dans le groupe constitué par le complément C3, la sérotransferrine, la vitronectine, la fibuline-1, la ficoline-3, le facteur H du complément, la chaîne lourde H4 de l'inhibiteur de l'inter-alpha-trypsine, l'apolipoprotéine L1, l'albumine sérique et les combinaisons de ceux-ci, le réactif pour déterminer le niveau d'expression étant choisi parmi le groupe constitué par:
(a) un anticorps, un aptamère ou un fragment de ceux-ci qui se lie spécifiquement à la protéine du biomarqueur; et
(b) un réactif approprié pour déterminer le niveau d'expression du biomarqueur au moyen d'un test de surveillance des réactions multiples (MRM), ledit réactif étant un peptide marqué par un isotope apte à être obtenu par digestion enzymatique de ladite protéine biomarqueur avec une enzyme digestive choisie dans le groupe constitué par la trypsine, la papaïne, l'endoprotéinase LysC, l'endoprotéinase ArgC, la staph aureus V8, la chymotripsine, l'Asp-N, l'Asn-C, la pepsine et l'endoprotéinase GluC.

12. Le kit selon la revendication 11, comprenant en outre un réactif pour déterminer le niveau d'expression d'un biomarqueur supplémentaire, ledit biomarqueur supplémentaire étant choisi dans le groupe constitué par la région C de la chaîne gamma-2 Ig, l'antithrombine-III, le complément C4A, le composant amyloïde P sérique et les combinaisons de ceux-ci, le réactif pour déterminer le niveau d'expression étant choisi dans le groupe constitué par:
a) un anticorps, un aptamère ou un fragment de ceux-ci qui se lie spécifiquement à la protéine du biomarqueur; et
(b) un réactif approprié pour déterminer le niveau d'expression du biomarqueur au moyen d'un test de surveillance des réactions multiples (MRM), ledit réactif étant un peptide marqué par un isotope apte à être obtenu par digestion enzymatique de ladite protéine biomarqueur avec une enzyme digestive choisie dans le groupe constitué par la trypsine, la papaïne, l'endoprotéinase LysC, l'endoprotéinase ArgC, la staph aureus V8, la chymotripsine, l'Asp-N, l'Asn-C, la pepsine et l'endoprotéinase GluC.

13. Le kit selon l'une quelconque des revendications 11 ou 12, dans laquelle lesdits réactifs sont des anticorps, ou des fragments de ceux-ci ayant la capacité de reconnaître l'antigène correspondant, qui se lient spécifiquement auxdits biomarqueurs.

14. Utilisation d'un kit comprenant un réactif pour déterminer le niveau d'expression d'au moins l'apolipoprotéine A-IV et éventuellement d'au moins un biomarqueur choisi parmi les biomarqueurs énumérés dans le tableau 1 pour le diagnostic d'un glaucome figurant parmi le glaucome primaire à angle ouvert et le glaucome à pseudo-exfoliation, le réactif pour déterminer le niveau d'expression étant choisi dans le groupe constitué par:
a) un anticorps, un aptamère ou un fragment de ceux-ci qui se lie spécifiquement à la protéine du biomarqueur; et
(b) un réactif approprié pour déterminer le niveau d'expression du biomarqueur au moyen d'un test de surveillance des réactions multiples (MRM), ledit réactif étant un peptide marqué par un isotope apte à être obtenu par digestion enzymatique de ladite protéine biomarqueur avec une enzyme digestive choisie dans le groupe constitué par la trypsine, la papaïne, l'endoprotéinase LysC, l'endoprotéinase ArgC, la staph aureus V8, la chymotripsine, l'Asp-N, l'Asn-C, la pepsine et l'endoprotéinase GluC.

15. Utilisation d'un kit selon la revendication 14, dans laquelle ledit réactif est un anticorps, ou un fragment de celui-ci ayant la capacité de reconnaître l'antigène correspondant, qui se lie spécifiquement audit biomarqueur.
